# EUROPEAN PATENT APPLICATION

(11) **EP 1 500 399 A1**
(43) Date of publication of application: **26.01.2005**
(21) Application number: 03291837.7
(22) Date of filing: 24.07.2003
(51) Int. Cl.: A61K 39/00, C07K 16/22, A61K 39/385, A61K 48/00, A61P 25/28, A61K 39/395

(54) **Active or passive immunization against proapoptotic neurotrophins for the treatment or prevention of neurodegenerative deseases**

(71) Applicant: INSTITUT PASTEUR, 75015 Paris (FR); Instituto de Investigaciones Biologicas Clemente Estable (I.I.B.C.E.), 11600 Montevideo (UY)
(72) Inventor: Barbeito, Luis Hector, 11400 CP Montevideo (UY); Estevez, Alvaro G., Dpt. of Physiologoy and Bioph., Birmingham, Alabama 35294-0005 (US); Beckman, Joseph S., Corvallis, Oregon 97331 (US); Pehar, Mariana, 11600 CP Montevideo (UY); Alzari, Pedro, 75015 Paris Cedex 15 (FR); Cassina, Maria Patricia, Depart. de Histologia, 11800 CP Montevideo (UY)
(74) Representative: Desaix, Anne

(57) **Abstract**

The present invention relates to novel methods for combatting cell degeneration or dysfunction resulting from neuroinflammatory conditions. The invention especially relates to the use, in the preparation of a medicament for the treatment of neurodegenerative disease associated with neuroinflammation, of an immunogenic compound which is capable of inducing an immune response against a proapoptotic neurotrophin, or an effective amount of a hapten combined with appropriate carriers and/or adjuvants to render the resulting combination capable of inducing an immune response against a proapoptotic neurotrophin. Also disclosed are compositions for the active or passive immunization against neuronal or glial cell apoptosis caused by neuroinflammantion as well as methods and means useful for said active or passive immunization.

## Description

The present invention relates to novel methods for combatting cell degeneration or dysfunction resulting from neuroinflammatory conditions. The invention especially relates to the use, in the preparation of a medicament for the treatment of neurodegenerative disease associated with neuroinflammation, of an immunogenic compound which is capable of inducing an immune response against a proapoptotic neurotrophin, or an effective amount of a hapten combined with appropriate carriers and/or adjuvants to render the resulting combination capable of inducing an immune response against a proapoptotic neurotrophin. Also disclosed are compositions for the active or passive immunization against neuronal or glial cell apoptosis caused by neuroinflammantion as well as methods and means useful for said active or passive immunization.

### Background of the Invention

**Neuroinflammation in neurodegenerative diseases.** Amyotrophic lateral sclerosis (in the following referred to as ALS, its abbreviation) is a neurodegenerative disease of the human motoneuron system which usually takes a lethal course within 3 to 5 years. The progressive decay of motor neurons are the cause of an increasing paralysis of the voluntary muscles, eventually leading to a total walking inability and the increasing paralysis of the respiratory musculature. Worldwide, the prevalence of this disease is 4 in 100,000 and its incidence is 1 in 100,000 inhabitants (Brooks et al, 1994). Since the original description of ALS in 1869, little progress has been made in understanding the etiology and pathogenesis of the most ALS cases and in consequence, no effective therapy has been developed to prevent or cure the disease.

Neuroinflammation in ALS is evidenced by the presence of reactive astrocytes and microglia expressing inflammatory markers. These cells surround upper and lower degenerating motor neurons and descending cortico-spinal tracts (Sasaki et al, 2000; Hirano, 1996; Kushner et al, 1991). Similarly, reactive neuroglia is found in spinal cord of transgenic mice and rats overexpressing ALS mutant SOD-1 (Sasaki et al, 2001; Alexianu et al, 2001; Howland et al, 2002; Bruijn et al, 1997), a well characterized animal model of the disease. Reactive astrocytes are known to upregulate the expression of inflammatory mediators and neurotrophic factors (Ridet et al, 1997), produce increased flows of nitric oxide and oxidants (Cassina et al, 2002), and downregulate glutamate transporters (Rothstein, 1996). NGF is upregulated in various neuropathologies in which reactive astrocytosis occurs (Crutcher *et al*, 1993, Gall *et al*., 1991; Lorez *et al*., 1989) and has been proposed as a mediator in tissue inflammation (Levi-Montalcini, 1996). However, there are wide gaps in information regarding whether astrocytic NGF is also upregulated in ALS or play a pathogenic role in the disease.

Alzheimer's disease (AD) is an irreversible, progressive brain disorder that occurs gradually and results in memory loss, behavioural and personality changes, and a decline in mental abilities. These losses are related to the death of brain cells and the breakdown of the connections between them. AD destroys neurons in parts of the brain that control memory, especially in the hippocampus and related structures. AD also attacks the cerebral cortex, particularly the areas responsible for language and reasoning. Two abnormal structures in the brain are the hallmarks of AD : amyloid plaques and neurofibrillary tangles. Plaques are dense, largely insoluble deposits of protein and cellular material outside and around the brain's neurons. Tangles are insoluble twisted fibers that build up inside neurons. In AD, amyloid plaques consist of largely insoluble deposits of beta amyloid a protein fragment of a larger protein called amyloid precursor protein intermingled with portions of neurons and with non-nerve cells such as microglia and astrocytes. Neuroinflammation in AD occurs around the amyloid plaques. It is characterized by the presence of reactive astrocytes and increased number of microglia (Pike et al., 1995; Beach et al., 1989; Schipper, 1996). Both cell types display inflammatory markers such a MHC-I and -II antigens, complement receptors and cytokine expression (McGeer et al., 1989). In addition, the levels of NGF in AD are elevated both in tissue and cerebrospinal fluid (Crutcher *et al*, 1993; Hock *et al*, 2000a; Hock *et al*., 2000b; Fahnestock *et al.*, 1996) and pro-NGF is the predominant form of NGF in AD (Fahnestock et al., 2001). Although NGF exerts trophic support of cholinergic neurons innervating the hippocampus and cerebral cortex, it may also cause apoptosis of hippocampal neurons expressing p75^{NTR} (Friedman et al., 2000; Brann et al., 2002; Troy et al., 2002). Thus, NGF may be implicated in the death of hippocampal neurons that change the ratio of TrkA/p75 ^{NTR} expression as a result of degenerative pathology in AD (Mufson et al., 1997; Hock et al., 1998).

Several other neurodegenerative diseases are characterized by the aggregation of tau into insoluble filaments in neurons and glia, leading to dysfunction and death. These disorders, which share some characteristics with AD but differ in several important aspects, are collectively called "fronto temporal dementia" and parkinsonism linked to chromosome 17. They are characterized by fronto-temporal atrophy with neuronal loss, grey white matter gliosis and superficial cortical spongiform. In addition, intraneuronal tau inclusions with the variable occurrence of glial inclusions are present (Kowalska, 2002). They are diseases similar to Parkinson's disease, some forms of amyotrophic lateral sclerosis (ALS), corticobasal degeneration, progressive supranuclear palsy, and Pick's disease, all characterized by abnormal aggregation of tau protein and a strong inflammatory reaction involving activated astroglia in the affected areas of the brain.

Other neurodegenerative diseases associated to neuroinflammation include prion diseases (such as kuru, Creutzfeld-Jacob disease and bovine spongiform encephalitis), Parkinson's disease and Huntington's disease. All involve deposits of abnormal proteins in the brain and activation of glial cells (Lefrancois *et al*., 1994; Liberski *et al.*, 2002; Renkawek *et al*., 1999; Schipper, 1996). Finally, neuroinflammation and gliosis plays a central pathogenic role in autoimmune disease affecting the CNS, such as multiple sclerosis (Massaro *et al*., 2002). Neuroinflammation also occurs following an ischemic or traumatic brain damage and is thought to substantially contribute to the permanent damage of brain tissue (Danton & Dietrich, 2003).

**Pathogenic role of neurotrophins in neuroinflammation.** Numerous experimental results indicate that increased neurotrophin production is associated with serious neurological diseases associated with neuroinflammation as described above. When activated, astrocytes produced increased amounts of neurotrophins, in particular NGF (Eddleston and Mucke, 1993; Ridet *et al*., 1997). Denervated muscle in ALS also produces increased amounts of neurotrophins including BDNF and NGF (Kust *et al*., 2002). In damaged or injured brains or spinal cords, increased neurotrophin production develops in parallel with expression of the p75^{NTR} receptor by brain cells (Beattie *et al*., 2002; Park *et al*., 2000). Such receptor is activated by neurotrophins or proneurotrophins and stimulates apoptotic death in brain cells including neurons or glial cells (for review see Hempstead, 2002; Dechant & Barde, 2002). Induction of p75 receptor expression has been observed in damaged neurons that are affected by ALS or multiple sclerosis pathology or by neurotrauma (Seeburger *et al*., 1993; Lowry *et al*., 2001; Chang *et al*., 2000; Roux *et al*., 1999).

Although these data suggest an involvement of neurotrophins in triggering cell death during neuroinflammation, it has been impossible so far to develop anti-apoptotic treatments for these conditions. Nevertheless, a number of therapies involving medication with a relatively unspecific action were attempted in order to suppress or at least modulate cell death occurring in neuroinflammation. However, such attempts remained without therapeutical success.

**Modulation of neuroinflammation by the immune system**. The immune system normally takes part in the clearing of foreign protein and particles in the organism but the inflammatory mediators such as neurotrophins associated with the above-mentioned diseases consist mainly of self-proteins, thereby escaping the role of the immune system. Further, neurotrophins are produced in the CNS which is normally separated from the immune system when the blood-brain-barrier is preserved. Thus, any immunotherapeutical approach or vaccine to produce antibodies against the proapoptotic neurotrophins in the CNS would be unsuccessful unless a disturbance of the blood-brain-barrier occurs as have been recognized in neuropathological conditions associated to inflammation of the CNS.

In the case of neurodegenerative diseases, however, a pathogenic mechanism based on increased production of pro-NGF by inflammatory astrocytes and the concomittant expression of p75^{NTR} in brain cells has not been disclosed so far.

### Description of the invention

The inventors have now found that it is possible to reduce neuronal or glial cell apoptosis occurring in a neurodegenerative disease, by administering an immunogenic derivative of neutrotrophin, enabling the production of antibodies directed against proapoptotic neurotrophin.

The invention thus concerns the use of a composition capable of inhibiting *in vivo* the binding of proapoptotic neurotrophin to p75^{NTR} receptor expressed by neuronal or glial cell, in the preparation of a medicament for inhibiting neuronal or glial cell apoptosis caused by neuroinflammation in an animal, especially in a mammal and more particularly in human.

As used herein the term "mammal" refers to animals of the mammal class of animals including human.

As used herein the term "neuroinflammation" is a general term that describes the characteristic changes occurring in brain or spinal cord tissue in response to degenerative, autoimmune, infectious, ischemic or traumatic damage. Neuroinflammation is characterized by activation and or proliferation of glial cells including astrocytes, microglia and oligodendrocytes in the site of injury.

In preferred embodiments of the invention, the term "neuroinflammation" refers to its occurrence in the context of a neurodegenerative disease, as it is observed in particular for the following neurodegenerative diseases: amyotrophic lateral sclerosis (ALS), Alzheimer's disease (AD), Parkinson's disease, Huntington's disease, Fronto temporal dementia, parkinsonism linked to chromosome 17 and prion diseases such as Kuru, Creutzfeld-Jacob disease, scrapie and bovine spongiform encephalitis.

As used herein, the term "neurotrophin" refers to the small family of dimeric secretory proteins that affect essentially all biological aspects of vertebrate neurons, including their survival, shape and function (for review see Huang et Reichardt, *Annu Rev Neurosci.* **24:** 677-736, 2001). In mammals, the neurotrophins are characterized by their ability to bind to and activate two structurally unrelated receptor types, the p75 neurotrophin receptor (hereafter referred to as p75^{NTR}) and the three members of the Trk receptor family of tyrosine kinases.

Neurotrophins are secreted in a precursor form, referred to as proneurotrophins. The precursor form can be cleaved by protease to produce the mature form. Unless otherwise specified, the term "neurotrophin" refers hereafter either to the precursor form of a neurotrophin or the mature form.

Neurotrophins are described more particularly by Scott et al., 1983; Ullrich et al., 1983; McDonald et al., 1991; McDonald et Blundell, 1991; Bradshaw et al., 1993; Hohn et al., 1990; Leibrock et al., 1989; Maisonpierre et al., 1990, Hallbook et al., 1991; Berkemeier et al., 1991

Preferably, the term "neurotrophin" refers to the the group consisting of NGF, BDNF, NT-3 and NT-4, pro-NGF, pro-BDNF.

As used herein, the term "proapoptotic neurotrophins" refers to endogenous secreted neurotrophins which bind to and activate p75^{NTR} receptor *in vivo,* thereby inducing neuronal or glial cell apoptosis.

According to the invention, a composition is considered to *in vivo* inhibit the binding of proapoptotic neurotrophin to p75^{NTR} receptor expressed in neuronal or glial cell if administration to a mammal of an effective amount of the composition can significantly reduce *in vivo* binding of proapoptotic neurotrophin to p75^{NTR} and subsequent neuronal or glial cell apoptosis. A reduction is considered significant if the reduction of binding and/or cell apoptosis is at least about 10%, preferably at least 50%, more preferably at least 80% and more preferably at least about 90%. Reduction of binding can be assayed by using competition displacement techniques known in the Art. Reduction of cell apoptosis can be assayed in an animal model of neurodegenerative disease such as transgenic mouse overexpressing mutant G93A-SOD1 gene.

According to a first object of the invention, inhibition of binding of neurotrophin to p75 ^{NTR} can be achieved by the administration of a composition comprising an effective amount of an immunogenic composition capable of inducing an immune response against a proapoptotic neurotrophin secreted by inflammatory cells such as astrocytes during neuroinflammation.

According to a second object of the invention, *in vivo* inhibition of binding of neurotrophin to p75^{NTR} can be achieved by the administration of a composition comprising an effective amount of a competitive inhibitor of said binding, or a molecule that binds to neutrotrophin or p75^{NTR}, thereby blocking the interaction between proapoptotic neurotrophin and p75^{NTR}.

According to the first object, the invention relates to an immunogenic composition that comprises an effective amount of an immunogenic compound which is capable of inducing an immune response against a proapoptotic neurotrophin, or an effective amount of a hapten combined with appropriate carriers and/or adjuvants to render the resulting combination capable of inducing an immune response against proapoptotic neurotrophin.

Said immunogenic composition can be used for the preparation of a medicament for inhibiting neuronal or glial cell apoptosis caused by neuroinflammation in an animal, as here above defined, and especially for the preparation of a medicament for the treatment of neurodegenerative disease associated with neuroinflammation.

As used herein the term "immune response against proapoptotic neurotrophin" means that the humoral immune response is sufficient to form antibodies that bind to one or more endogenous proapoptotic neurotrophins, thus neutralizing their ability to activate p75^{NTR}, in neuronal or glial cells.

In a specific embodiment of the invention, said immunogenic compound or hapten comprises or essentially consists of a neurotrophin or a fragment of a neurotrophin which can be rendered immunogenic when combined with appropriate carriers and/or adjuvants.

Naturally, derivatives of said neurotrophin or fragments thereof, which are modified, for example, by amino acid substitution, deletion and/or addition but having substantially the same immunological properties as the native neurotrophin or the native corresponding fragment of neurotrophin can be used alternatively.

In a preferred embodiment, said modification by amino acid substitution, deletion and/or addition does not affect the tertiary structure of the resulting modified neurotrophin or neurotrophin fragment as compared to the native one from which it derives. For example, the modification consists of conservative substitution of amino acid residues.

According to a specific embodiment, the functional derivatives exhibit at least 70% identity, preferably 80% identity and more preferably 90% identity when compared with the corresponding sequence of the native neurotrophin or neurotrophin fragment from which it derives.

In this comparison to determine percent identity, the sequences should be aligned for optimal comparison. For example gaps can be introduced in the sequence of a first amino acid sequence for optimal alignment with the second amino acid sequence. Optimal alignment for determining a comparison window may be conducted by the local homology algorithm of Smith and Waterman (1981), by the homology alignment algorithm of Needleman and Wunsch (1972), by the search for similarity via the method of Pearson and Lipman (1988) or by computerized implementations of these algorithms (GAP, BESTFIT, FASTA and TFASTA in the Wisconsin Genetics Software Package Release 7.0, Genetic Computer Group, 575, Science Drive, Madison, Wisconsin). The best aligment (i.e., resulting in the highest percentage of identity over the comparison window) generated by the various methods is selected.

Advantageously, said amino acid substitution, deletion and/or addition is selected in order to increase the immunogenicity of the modified neurotrophin or neurotrophin fragment as compared to the native one.

Such derivatives will be referred hereafter as functional derivatives of neurotrophin.

Neurotrophin fragments and their functional derivatives as defined above will be referred hereafter as neurotrophin fragments.

Neurotrophins fragments which are used according to the invention are preferably polypeptides from 10 to 100 amino acids, more preferably, from 20 to 70 amino acids, and most preferably from 20 to 50 amino acids.

In a preferred embodiment, a neurotrophin fragment is effective to induce antibodies directed to a neoepitope, *e.g.*, an epitope that is revealed by proteolytic cleavage, but that is not present on the precursor form of the neurotrophin. Such neurotrophin fragments are preferred since they may be less likely to induce an autoimmune response in the patient.

The neurotrophin or neurotrophin fragments may be fused to other heterologous polypeptidic sequences. Especially, such neurotrophin or neurotrophin fragments can be coupled to one single or a few heterologous epitopes to specifically promote humoral immunological responses.

Neurotrophin or neurotrophin fragments used according to the invention may also contain additional signals for their efficient stability, secretion and/or purification.

According to one specific embodiment, said neurotrophin or neurotrophin fragment comprises a mature form of a neurotrophin, preferably selected from the group consisting of NGF and BDNF, or a functional derivative thereof.

According to another embodiment, said neurotrophin or neurotrophin fragment comprises a precursor form of a neurotrophin, such as those selected from the group consisting of proNGF and proBDNF, or a functional derivative thereof.

Said neurotrophin fragment may for example comprises a fragment of a precursor of mature neurotrophin, said fragment comprising at least part of its amino acid sequence which is not comprised in the corresponding mature form of the neurotrophin, such as the preprodomain of NGF.

The neurotrophin or neurtrophin fragments can be selected among the group consisting of NGF, BDNF, NT-3 and NT-4, pro-NGF, pro-BDNF or fragments thereof.

Neurotrophin fragments can be selected for example among the fragments of neurotrophins comprising the functional domain involved in binding to p75^{NTR} receptor.

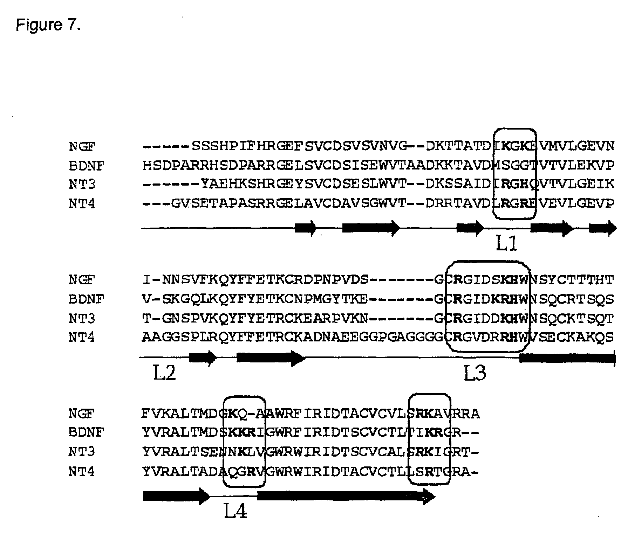

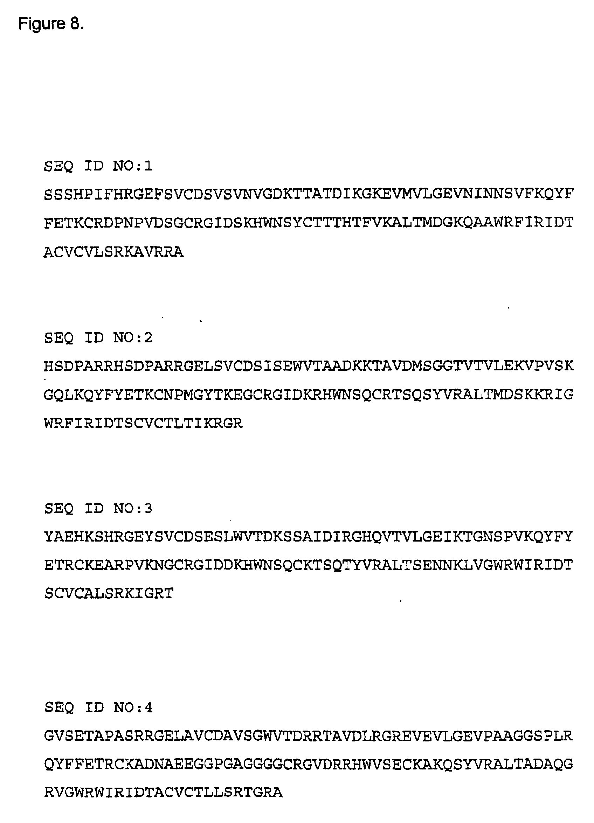

These domains are shown for example in figure 7, for NGF, BDNF, NT3 and NT4 neurotrophins.

In a specific embodiment of the invention, said neurotrophin fragment comprised in the immunogenic composition according to the invention, is a peptide or polypeptide comprising at least 6 consecutive amino acids of one the following sequences:
a. an immunogenic or hapten fragment of any of SEQ ID NOs 1-4,
b. an immunogenic or hapten fragment of SEQ ID NO:1 comprising one of the following sequences: IKGKE (SEQ ID NO:5), CRGIDSKHW (SEQ ID NO:6), GKQA (SEQ ID NO:7) and SRKAV (SEQ ID NO:8),
c. an immunogenic or hapten fragment of SEQ ID NO:2 comprising one of the following sequences: MSGGT (SEQ ID NO:9), CRGIDKRHW (SEQ ID NO:10), SKKRI (SEQ ID NO:11), TIKRG (SEQ ID NO:12),
d. an immunogenic or hapten fragment of SEQ ID NO:3 comprising one of the following sequences: IRGHQ (SEQ ID NO:13), CRGIDDKHW (SEQ ID NO:14), NNKLV (SEQ ID NO:15), SRKIG (SEQ ID NO:16),
e. an immunogenic or hapten fragment of SEQ ID NO:4 comprising one of the following sequences: LRGRE (SEQ ID NO:17), CRGVDRRHW (SEQ ID NO:18), AQGRV (SEQ ID NO:19), LSRTG (SEQ ID NO:20).
f. a peptide exhibiting at least 70% identity, preferably 80% identity and more preferably 90% identity with one of the immunogenic or hapten fragment defined in a.-e., said peptide retaining the same immunological properties as the native one from which it derives.

When a neurotrophin fragment comprised in the immunogenic composition of the invention is not immunogenic per se, but is a hapten, it can be made immunogenic by coupling the hapten to a carrier molecule such as bovine serum albumine (BSA). Other preferred carriers include immunoglobulin molecules, thyroglobulin, ovalbumin, tetanus toxoid, keyhole limpet hemocyanin or lipid moieties. Various carrier molecules and methods for coupling a hapten to a carrier molecule are well-known in the Art (Bioconjugation. Protein coupling techniques for the biomedical sciences". 364-482, Ed. Aslam M. & Dent A. Mcmillan Reference LTD, UK, 1998).

The immunogenic composition of the invention is formulated for administration to a patient suffering of neuronal or glial cell apoptosis caused by neuroinflammation, and especially to a patient suffering of neurodegenerative disease.

The immunogenic composition of the invention can be administered alone or in combination with an acceptable vehicle, including water, saline, glycerol, ethanol, etc. The compositions can also be administered in combination with other therapeutical agents, especially useful for the treatment of neuronal or glial cell apoptosis caused by neuroinflammation, and/or useful for the treatment of neurodegenerative diseases. Typically, the compositions are prepared as an injectable composition, either as a liquid solution or a suspension. However, solid compositions suitable for solution or suspension in liquid vehicles prior to injection can also be prepared.

An effective amount of the immunogenic compound or hapten, useful for inducing an immune response against proapoptotic neurotrophin can be determined on a case-by-case basis.

According to a preferred embodiment, the immunogenic composition is effective to produce an immune response that is characterized by a serum titer of at least 1:1000 with respect to the neurotrophin antigenic determinant against which the immune response is directed. In yet a further preferred embodiment, the serum titer is at least 1:5000 with respect to the neurotrophin component. According to a specific embodiment, the immune response induced by the immunogenic composition is characterized by a serum amount of immunoreactivity corresponding to more than four times higher than a serum level of immunoreactivity measured in a pre-treatment control serum sample. This latter characterization is particularly appropriate when serum immunoreactivity is measured by ELISA techniques, although it can apply to any relative or absolute measurement of serum immunoreactivity.

For example, an effective amount of the active ingredient is comprised between 0,5 µg and 2000 µg.

The immunogenic composition is preferably formulated as a vaccine. Such vaccine composition includes generally specific excipients and preferably adjuvants, to enhance the immune response.

For example, an adjuvant can be a particulate or non-particulate adjuvant. A particulate adjuvant usually includes, without limitation, aluminium salts, calcium salts, water-in-oil emulsions, oil-in-water emulsion, immune stimulating complexes (ISCOMS) and ISCOM matrices (US No. 5,679,354), liposomes, nano- or micro-particles, proteosomes, virosomes, stearyl tyrosine, and gamma-inulin. A non-particulate adjuvant usually includes, without limitation, muramyl dipeptide (MDP) and derivatives, e.g., treonyl MDP or murametide, saponins, e.g., Quil A and QS21, lipid A or its derivative 4'monophosphoryl lipid A (MPL), various cytokines including gamma-interferon and interleukins 2 or 4, carbohydrate polymers, diethylaminoethyl dextran and bacterial toxins, such as cholera toxin. Adjuvants formulation designed to maximize specific immune response can also be used.

In preferred embodiments, adjuvants are selected among the group consisting of aluminium hydroxide, aluminium phosphate, MPL1M, QS-21 or incomplete Freund's adjuvant. According to a specific embodiment, such immunogenic compositions may include a plurality of immunogenic compounds effective to induce an immune response against at least two different neurotrophin antigens in a patient.

The invention also pertains to the method for treating or preventing neuronal or glial cell apoptosis caused by neuroinflammation, comprising the administration to a patient suffering of neuronal or glial cell apoptosis caused by neuroinflammation, of a composition capable of inhibiting *in vivo* the binding of proapoptotic neurotrophin to p75^{NTR} receptor expressed by neuronal or glial cell as defined above. Especially, the invention relates to a method for treating or preventing neuronal or glial cell apoptosis caused by neuroinflammation, comprising the administration to a patient suffering of neuronal or glial cell apoptosis caused by neuroinflammation, of an immunogenic composition capable of inducing an immune response directed against proapototic neurotrophin.

Especially, the immunization regimens may include administration of the immunogenic composition, in multiple dosages, for example over a 6 month period for an initial immunization followed by booster injections at time intervals, for example 6 weeks period, according to methods well known in the Art, or according to patient need, as assessed by measuring immunological response.

According to the second object of the invention, an example of an active ingredient which inhibits the binding of neurotrophin to p75^{NTR} receptor is a compound that binds to the recognition domain of p75^{NTR}, thereby competitively inhibiting the binding of neurotrophin to p75^{NTR}, or a molecule that binds to neurotrophin or p75^{NTR}, thereby blocking the interaction between proapoptotic neurotrophin and p75^{NTR}.

More specifically, said active ingredient is an antagonist of said neurotrophin. Antagonists of neurotrophins may be selected among fragments of said neurotrophin or their derivatives having at least 70% identity, preferably at least 80% identity and more preferably, at least 90% identity with the native fragment.

Compounds that block the interaction of proapoptotic neurotrophin to p75^{NTR} can also be advantageously selected among the antibodies directed against a neurotrophin or a fragment thereof, capable of down-regulating proapoptotic activity of endogenous neurotrophins secreted by astrbcytes during neuroinflammation, referred hereafter as blocking antibodies.

inhibition of binding of neurotrophin to p75^{NTR} can be thus achieved by the administration of an effective amount of a composition comprising blocking antibodies capable of down-regulating proapoptotic activity of endogenous neurotrophins secreted by astrocytes during neuroinflammation. The blocking of binding of the neurotrophins to their receptor inhibits neuronal apoptosis caused by neuroinflammation, thereby preventing or delaying the neurodegenerative process.

According to a still further related aspect, the invention provides a method of determining the prognosis of a patient undergoing treatment for a neuroinflammatory disorder. Here, patient serum amount of immunoreactivity against a neurotrophin component characteristic of the selected disorder is measured, and a patient serum amount of immunoreactivity of at least four times a baseline control level of serum immunoreactivity is indicative of a prognosis of status with respect to the particular neuroinflammatory disorder.

The invention thus also concerns a composition comprising an effective amount of blocking antibodies, in combination with an acceptable vehicle for *in vivo* administration, said composition being useful to treat or reduce neuronal or glial cell apoptosis caused by neuroinflammation.

Naturally occurring antibodies are obtained by a process comprising a step of immunization of a mammal with the neurotrophin or neurotrophin fragment.

As used herein, the term "blocking antibodies" also include non-naturally occurring antibodies, such as for example, single chain antobodies, chimeric antibodies, bifunctional antibodies and humanized antibodies, as well as antigen-binding fragments thereof. Such non-natural occuring antibodies can be constructed using phase peptide synthesis, or can be produced recombinantly, or can be obtained, for example, by screening combinatorial libraries. Other methods of making, for example, chimeric, humanized, CDR-grafted, single chain, and bifunctional antibodies are well known in the Art.

The term "blocking antibody" refers especially to fragment or derivative of an antibody retaining the same binding affinity towards neurotrophin or a fragment thereof as the native antibody from which it derives. A derivative is more preferably a polypeptide exhibiting at least 70% identity, preferably at least 80% identity and more preferably at least 90% identity with a native fragment of the antibody from which it derives.

According to a specific embodiment, the composition may include a combination of antibodies that bind at least to two different neurotrophins.

In general, an effective amount of blocking antibodies correspond to a serum amount of immunoreactivity against the target neurotrophin component that is at least about four times higher than a serum level of immunoreactivity against the same component measured in a control serum sample.

Blocking antibodies can either be monoclonal or polyclonal antibodies.

In a specific embodiment, said blocking antibody is a monoclonal antibody raised against a specific epitope contained in a neurotrophin. Monoclonal antibodies can be obtained especially by the usual method developped by Kohler and Milstein, 1975.

Furthermore, humanized monoclonal antibodies can be prepared by cloning the genes encoding the heavy and light chains of monoclonal antibodies produced by hybridomas, these sequences being *in vitro* manipulated and reintroduced into secreting cells, such as lymphoid cells or others, after their insertion in appropriate expression vectors. Monoclonal antibodies can thus be obtained with variable regions of mice or rats and constant human regions.

Alternatively, blocking antibodies can be isolated from a polyclonal serum obtainable by immunizing a mamma( with an immunogenic compound or composition capable of inducing an immune response against a neurotrophin as described above.

The blocking antibodies can be raised specifically against the mature form of neurotrophin. The invention thus also concerns a composition for the prevention and/or the treatment of neurodegenerative diseases associated with neuroinflammation, comprising an effective amount of an antibody directed against a mature form of a neurotrophin, and preferably NGF or BDNF.

The invention also relates to the use of an antibody directed against a mature form of a neurotrophin in the preparation of a composition for the prevention and/or the treatment of neurodegenerative diseases associated with neuroinflammation.

The invention also concerns the use of an antibody directed against a neurotrophin or a fragment thereof or a functional derivative thereof, in the preparation of a drug for preventing neuronal cell death caused by neuroinflammation.

The compositions of the invention as described above can be administered according to any pharmaceutically effective route.

Possible administration routes include peritoneal, oral, intranasal, subcutaneous, intramuscular, topical or intravenous administration.

The active ingredients of the compositions including the immunogenic or hapten compounds or blocking antibodies can be prepared according to any appropriate means known in the Art.

When the active ingredients used in the composition according to the invention are selected among specific polypeptides, these polypeptides can be either isolated from natural cells secreting such polypeptides or can be chemically synthesized according to usual methods in the Art. Polypeptides can be advantageously prepared by expressing a nucleic acid encoding said polypeptide in an appropriate host cell and recovering the expressed polypeptides.

Another object of the invention thus relates to a nucleic acid encoding neurotrophin fragments as defined above, or a nucleic acid encoding blocking antibodies as defined above.

More preferably, the invention is directed to a nucleic acid encoding a specific hapten fragment derived from SEQ ID NOs 1-4 as described above.

The invention also concerns a vector comprising a nucleic acid as defined above. As used herein, the term "vector" refers to any appropriate structure allowing the introduction of said nucleic acid sequence in a host cell, and the replication of said nucleic acid in the host cell and optionnaly, expression of said nucleic acid in said host cell.

Preferably, said vector is capable of autonomous replication in a mammalian cell. Examples of such vectors include a plasmid, a phage, a cosmid, a minichromosome, and a virus. The vectors of the invention may also comprise appropriate sequences for secretion of the translated protein out of the host cell.

In another specific embodiment of the invention, said polypeptide useful for providing an immune response against an apoptotic neurotrophin is synthesized *in vivo.* The vector is thus selected among appropriate vectors used for gene therapy treatment. As used herein, the term "gene therapy treatment" refers either to direct delivery of the therapeutic nucleic acid into a patient or indirect *ex vivo* gene therapy (*i.e.*, cells are first transformed with the nucleic acid *in vitro* and then transplanted into the patient). Vectors for gene therapy treatment include for example defective or attenuated retroviral or other viral vectors as described in US patent 4,980,286. The various retroviral vectors that are known in the Art are also described for example in Miller *et al.* (1993) which have been modified to delete those retroviral sequences which are not required for packaging of the viral genome and subsequent integration into host cell DNA. Vectors that target neuronal or glial cells are preferred.

The invention further relates to the host cells transformed by the vectors above-defined. In a specific embodiment, the host cells are selected among the group consisting of bacterial cells, such as *E*. *coli,* eucaryotic cells, such as fungi, insects cells, Drosophila or plant cells. More preferably, host cells are selected among mammalian cells, and more particularly mammalian cell lines including CHO, HeLa, C127, 3T3, HepG2 and L(TK)- cells.

The following experimental part shows the results obtained by the expression of NGF pro-forms in the spinal cords of mice carrying the G93A SOD1 mutation and reactive astrocytes in culture, as well as the effect of neurotrophin autoimmunization on paralysis onset and survival in a transgenic mouse model of ALS.

### Description of the figures

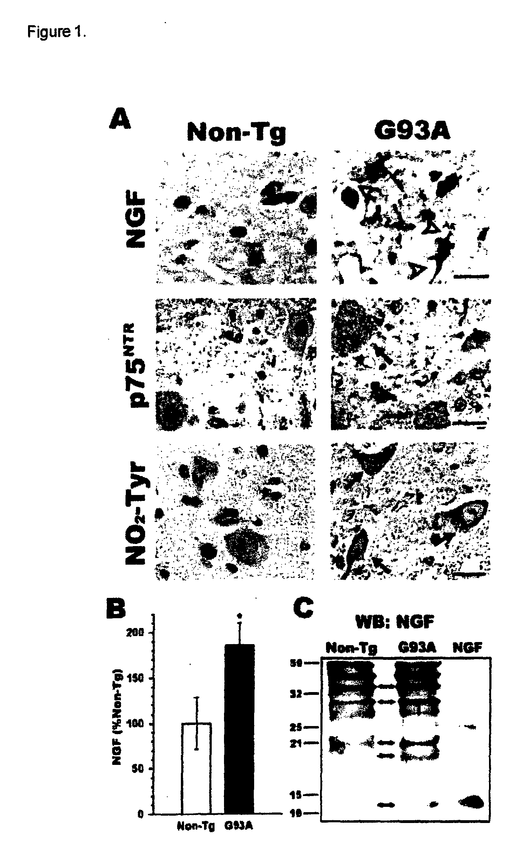

**Fig. 1. Up-regulation of NGF and proNGF expression in G93A SOD-1 transgenic mice.**
   **A**. Tissue sections from the spinal cords of symptomatic 90-day-old G93A transgenic mice and non-transgenic littermates (Non-Tg) were immunostained with the indicated antibodies and counterstained with hematoxylin.
      **Upper row**: representative aspect of the neuropil of the anterior horn following immunostaining with anti-NGF-β polyclonal antibody (Chemicon). NGF immunoreactivity was found in non-neuronal cells with typical astrocyte morphology. Arrowheads indicate astrocytic processes wrapping around vacuoles. Similar results were obtained with anti-NGF-β monoclonal antibodies (Chemicon; not shown). Scale bar: 15 µm.
      **Middle row:** p75^{NTR} immunoreactivity using a polyclonal antibody (Chemicon) following the Envision amplification protocol (Dako). p75^{NTR} was mainly localized in a population of motor neurons (arrows) in transgenic G93A mice. Similar results were obtained with other antip75 antibodies (Advanced Targeting Systems, not shown). Scale bar: 20 µm.
      **Lower row**: Nitrotyrosine immunoreactivity was significantly increased in the neuropil and large motor neurons in transgenic mice (arrows). Scale bar: 20 µm.
   **B.** ELISA determination of NGF levels in the spinal cord of 90-day-old transgenic mice. Data are expressed as percentage of NGF levels in non-transgenic
      littermates (100%= 21.2 ± 6.4 pg/mg protein). * p<0.05 with respect to nontransgenic.
   **C.** Western blot of lumbar spinal cord extracts from G93A symptomatic mice or non-transgenic littermates (non-Tg) (50 µg) using an anti-NGF polyclonal antibody (Chemicon AB1526SP). Purified NGF (0.5 µg) from Harlan was used as a control. NGF bands were not detected following incubation of NGF antibodies with an excess of purified NGF. Arrows indicate immunoreactive bands up-regulated in symptomatic G93A spinal cord extracts.

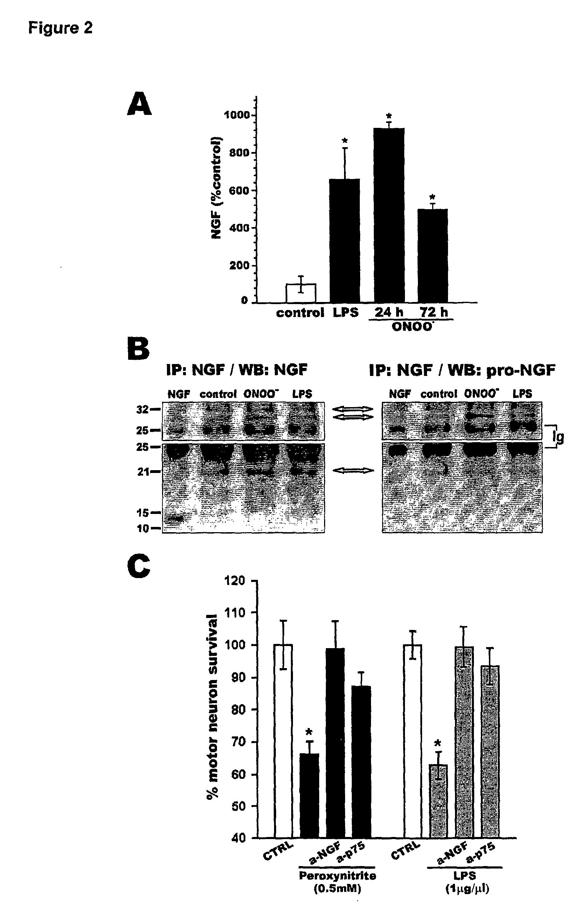

**Fig. 2. ProNGF secretion by reactive astrocytes and p75-dependent motor neuron apoptosis in co-cultures.**
   **A.** ELISA determination of NGF levels in the conditioned media from untreated cultured spinal cord astrocytes (control), following 24 h stimulation with LPS (1µg /ml) or 24 h and 72 h after exposure to 0.5 mM peroxynitrite (ONOO⁻). Data are expressed as percentage of NGF levels in control (100% = 10.9 ±2.1 pg/ml) *Significantly different from control (p<0.05).
   **B.** NGF is mainly secreted as its precursor forms. Conditioned media (24 h) were immunoprecipitated with rabbit anti-NGF polyclonal antibody (Santa Cruz) and analyzed by Western blot using with anti-proNGF polyclonal antibodies. The membranes were stripped and reprobed with anti-mature NGF polyclonal antibody (Chemicon). 2.5 µg of purified NGF (Harlan) was immunoprecipitated as an internal control. Arrows indicate secreted proNGF immunoreactive bands upregulated in reactive astrocytes. To show in detail the low molecular weight NGF bands, it was necessary to expose the lower part of the membrane three times longer than the upper part. Ig indicates the position of the immunoglobulin light chain. **C.** p75-dependent motor neuron death induced by reactive astrocytes. Purified motor neurons from E15 rat embryos were plated on astrocyte monolayers previously stimulated with vehicle (CTRL), LPS or peroxynitrite and motor neuron survival was determined after 72 h. Reactive astrocyte-mediated death as prevented by anti-NGF (a-NGF, 1:500 Chemicon AB1526SP) or antip75 (a-p75, 1:100, Chemicon) blocking antibodies but not by non-immune serum. Similar results were obtained using a different set of blocking antibodies to NGF (1:500 Chemicon MAB5260Z) or p75 (1:200, Advanced Target Systems). Data are expressed as percentage of control, mean ± SD. * significantly different from control (p<0.05).

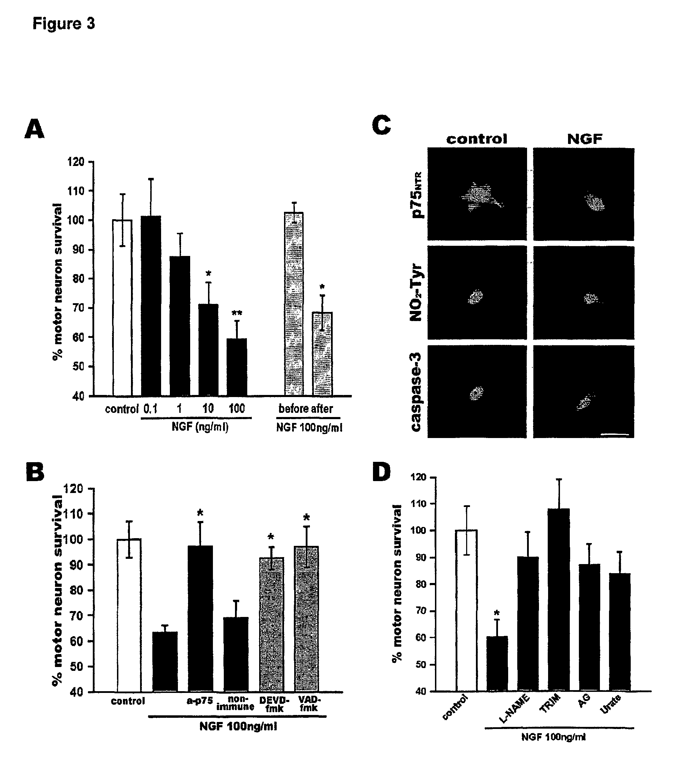

**Fig. 3. Exogenous NGF induces motor neuron apoptosis in co-cultures.**
   **A**.Co-cultures were treated with NGF (0.1-100 ng/ml) and motor neuron survival was determined after 72 h. Gray bars represent the percentage of neuronal survival when 100 ng/ml NGF was added for 24 h to astrocytes alone before motor neuron plating (before) or to co-cultures 24 h after neuronal plating (after).
      Data are expressed as percentage of control, mean ± SD.*Significantly different from control (p<0.05).
   **B.** Fluorescence micrographs showing immunoreactivity for p75^{NTR} (red), nitrotyrosine (red, NO2-Tyr) and cleaved caspase-3 (red) in cocultures treated for 24 h with vehicle (control) or 100 ng/ml NGF. Motor neurons were identified by Islet-1/2 homeoprotein-immunoreactivity (yellow/green). Scale bar: 20 µm.
   **C**. Blocking antibodies to p75^{NTR} (a-p75, 1:100, Chemicon) and caspase inhibitors DEVD-fmk (10 µM) or VAD-fmk (10 µM) prevented NGFinduced motor neuron death. Antibodies to p75^{NTR} and non-immune serum were added once immediately after motor neuron plating, and caspase inhibitors every 24 hours thereafter. Data are expressed as percentage of control (mean ± SD).*significantly different from NGF (p<0.05).
   D. NOS inhibitors prevent NGFinduced motor neuron apoptosis. Cocultures were treated with vehicle (control) or NGF in the presence of L-NAME (1mM), TRIM (10 µM), LNIL (10 µM) or urate (200 µM) and motor neuron survival was determined after 72 h. Motor neuron death was also significantly prevented by NPLA (10µM; 110.4±9.2 %) and aminoguanidine (50 µM; 87.0±8.0 %). Data are expressed as percentage of control, mean ± SD. * Significantly different from control (p<0.05).

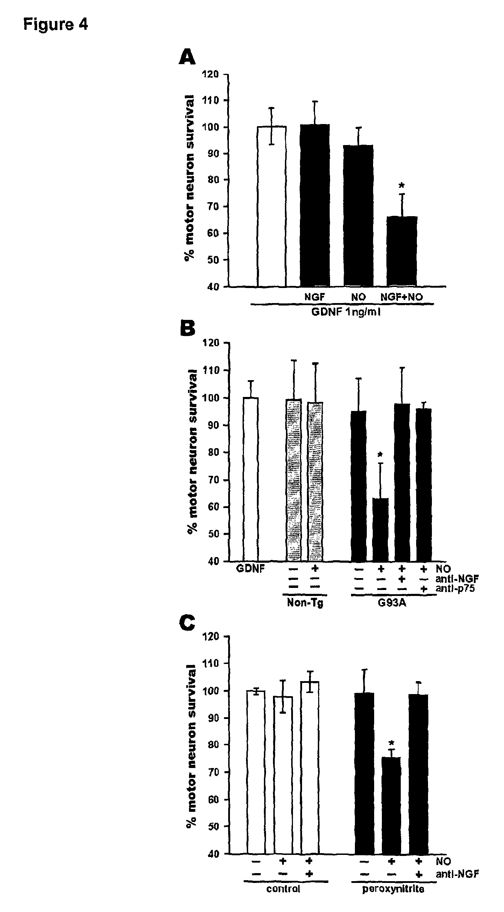

**Fig. 4. NGF-dependent apoptotic activity present in degenerating spinal cords or conditioned media from reactive astrocytes.**
   Pure motor neuron cultures maintained with GDNF (1 ng/ml) alone or in the presence of NOC-18 (10µM, NO) were exposed to: **A**. exogenous NGF (100 ng/ml); **B**. spinal cord extracts (0.5 µg prot/ml) from G93A mice or non-trangenic littermates (Non-Tg);or **C**. astrocyte-conditioned media obtained 24 h after exposure to vehicle (control) or peroxynitrite (0.5 mM). Motor neuron survival was determined after 48 h. The death mediated by the spinal cord extracts or conditioned media was significantly attenuated by anti-NGF (1:500 Chemicon AB1526SP) or anti-p75 (1:100, Chemicon) blocking antibodies but not by non-immune serum. Similar results were obtained using a different set of blocking antibodies to NGF (1:500 Chemicon MAB5260Z) or p75 (1:200, Advanced Target Systems). Data are expressed as percentage of GDNF, mean ± SD. * Significantly different from GDNF (p<0.05).

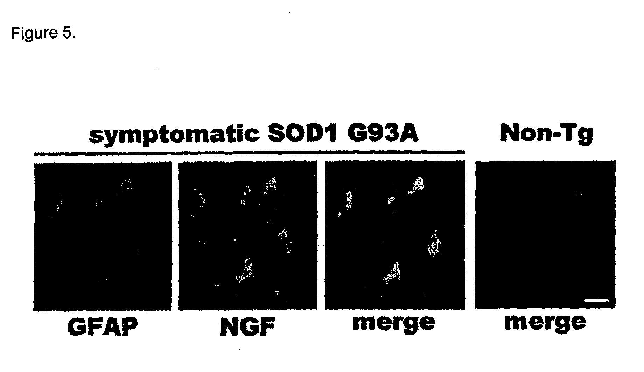

**Fig. 5. Reactive astrocytes express NGF.**
   Double immunofluorescence staining of GFAP (red) and NGF (green) and co-localization of both antigens (yellow in merge) in the ventral horn of symptomatic G93A mice spinal cords. In comparison, non-transgenic littermates (Non-Tg) showed staining for neither GFAP nor NGF. Nuclei were visualized with DAPI in merged images. The lumbar spinal cords were dissected from 90-day-old transgenic mice and nontransgenic littermates after perfusion as described *in Methods.* The tissue was post-fixed overnight at 4°C in 4 % paraformadehyde in 0.1 M phosphate buffer (pH 7.4), cryoprotected in 30% sucrose and transversal 6 mm sections were obtained using a cryostat. Sections were mounted on super-frost glass slides and stored at -80°C until use. Each section was air-dried and processed for immunofluorescence as described in *Methods.* Primary antibodies were anti-NGF-β polyclonal (1:450; Chemicon) and anti-GFAP Cy3 conjugated (1:400 Sigma). Secondary antibodies were biotin-labeled goat anti-mouse (1:125,Jackson) followed by TSATM Biotin System (PerkinElmer) using Fluoresceinconjugated streptavidin (1:100, Vector). Scale Bar: 20 µm.

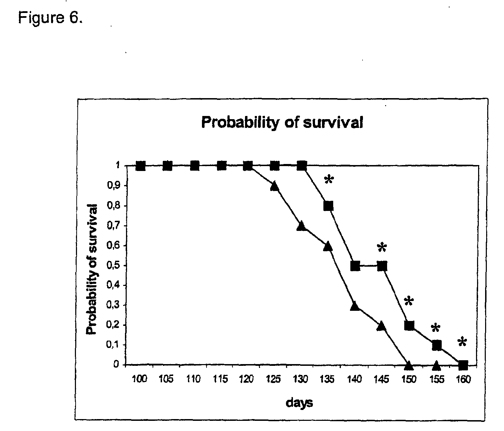

**Fig.6. Systemic immunization against NGF delayed disease onset and death in G93A SOD-1 transgenic mice.**
   G93A female mice were autoimmunized against NGF (N=10 in each group, squares in the graph). Each received two subcutaneous injection of 25 µg of 2.5S mouse NGF (Harlam, USA) in 0.1 ml of a suspension of aluminum phosphate used as adjuvant. The first injection was given at age of 40-50 days. The second injection of 2.5S NGF (50 µg) in the same adjuvant was given 3 weeks later. Adjuvant-injected G93A female mice of the same age served as control animals for survival tests (triangues in the graph). Note the significantly increased in survival in the group of mice receiving NGF immunization.
**Fig. 7: Synthetic peptides for preventing neuronal or glial cell apoptosis by active immunization.**
   The figure 7 shows the sequence alignment of various neurotrophins (NGF: nerve growth factor, BDNF: brain-derived neurotrophic factor, NT3: neurotrophin-3, NT-4: neurotrophin-4). Secondary structure elements are shown in green. Residues important for p75^{NTR} binding are shown in bold, in loops L1, L3, and L4, and in the C-terminus of the protein.

### Examples

### Materials and Methods

**Materials.** Transgenic mice for G93A human SOD1 strain B6SJLTgN (SOD1-G93A)1Gur (Gurney *et al*., 1994), and a control strain were purchased from Jackson Lab. Culture media and serum were obtained from Gibco-Invitrogen, mouse NGF(2.5S) from Harlan, DEVD-fmk and VAD-fmk from Calbiochem (San Diego), and Fe(III)-tetra (carboxyphenyl) porphyrin (FeTCPP) from Frontier Scientific (Utah). Nitro-L-arginine-methyl ester (L-NAME), [N5[Imino(propylamino)methyl]-Lornithine;Nù-Propyl-L-arginine] (NPLA), [1-(2-Trifluoromethylphenyl)imidazole](TRIM); and L-N₆-(1-Iminoethyl)-lysine (LNIL) were from Alexis (San Diego). All other reagents were from Sigma, unless otherwise specified.

**Cell cultures and treatments**. Primary astrocyte cultures were prepared from the spinal cords of raths aged 1-2 days according to the procedures of Saneto and De Vellis (1987), with minor modifications (Cassina *et al*., 2002). Astrocytes were plated at a density of 2x10⁴ cells/cm² and maintained in DMEM supplemented with 10% fetal bovine serum, HEPES (3.6 g/l), penicillin (100 IU/ml) and streptomycin (100µg/ml). The astrocyte monolayers were >98% pure as determined by GFAP immunoreactivity and were devoid of OX42-positive microglial cells.

Motor neuron cultures were prepared from rat embryonic spinal cords (E15) by a combination of metrizamide gradient centrifugation and immunopanning with the monoclonal antibody IgG192 against p75^{NTR} (Henderson *et al*., 1995). For co-culture experiments, motor neurons were plated on astrocyte monolayers at a density of 300 cells/cm² and maintained in L15 medium supplemented with 0.63 mg/ml bicarbonate, 5 µg/ml insulin, 0.1 mg/ml conalbumin, 0.1 mM putrescine, 30nM sodium selenite, 20 nM progesterone, 20 mM glucose, 100 IU/ml penicillin,100 µg/ml streptomycin, and 2% horse serum. Drugs and blocking antibodies were added 3h after plating at the indicated concentrations.

Purified motor neuron cultures were plated at a density of 300 cells/cm² in dishes precoated with polyornithinefaminin and maintained in Neurobasal medium supplemented with 2% horse serum, 25 mM L-glutamate, 25 µM β-mercaptoethanol, 0.5 mM L-glutamine, and 2% B-27 supplement (Gibco-Invitrogen). Blocking antibodies were added 3h after plating at the indicated dilutions. The generation of a low steady state concentration (<50 nM) of nitric oxide was generated by the spontaneous deassociation of 10 µM DETA-NONate (Cayman).

To assess the pro-apoptotic activity of conditioned media, astrocyte monolayers were incubated for 24 h in Neurobasal medium supplemented as described above, and that medium immediately used to replace the medium of pure motor neuron cultures established 24 h before. Blocking antibodies and DETA-NONOate were added to the conditioned media and motor neuron survival determined after 48h.

To assess the pro-apoptotic activity in spinal cord extracts, lumbar cords were dissected from 90 day-old G93A mice or non-transgenic littermates over ice under sterile conditions. To 100 mg of tissue were added 0.4 ml of PBS containing 3 mM EGTA, 1 mM EDTA, 0.5 µg/ml aprotinin, 0.5 µg/ml pepstatin and 0:1 mM PMSF at 0°C, and the tissue was then homogenized under sterile conditions. Homogenates were centrifuged at 40,000g for 1 hr and the clear supernatants collected and kept at -80°C until used. Aliquots were added to motor neuron cultures to reach a final protein concentration of 0.5 µg/ml. In all cases, blocking antibodies and drugs were added 3 h after motor neuron plating. The generation of a steady state concentration (<50 nM) of nitric oxide was obtained by the spontaneous disassociation of 10 µM NOC-18 (Dojindo, Gaithersburg).

**Treatments with peroxynitrite and LPS.** Peroxynitrite was generously provided by Dr. Rafael Radi (UDELAR, Uruguay) and its concentration determined spectrophotometrically at 302 nm (e=1700 M⁻¹.cm⁻¹). Confluent astrocyte monolayers were washed with Dulbecco's phosphate-buffered saline (PBS), supplemented with 0.8 mM MgCl₂, 1 mM CaCl₂, and 5 mM glucose, and then incubated in 1 ml of 50 mM Na₂HPO₄, 90 mM NaCl, 5 mM KCI, 0.8 mM MgCl₂, 1 mM CaCl₂, and 5 mM glucose, pH 7.4. Finally, three additions of 5 µL bolus of peroxynitrite were made to reach the final concentration of 0.5 mM. Five minutes after peroxynitrite exposure, the buffer was removed and replaced with L15 medium, supplemented as described above. In co-culture experiments, motor neurons were plated 1 h after peroxynitrite exposure. Control treatments were performed using diluted NaOH (vehicle) or decomposed peroxynitrite (Estevez *et al*., 1998). LPS (from E. coli 026-B6 Cat. No L2654 Sigma) was directly applied to astrocyte monolayers 24 h before and immediately after plating of motor neurons in coculture experiments.

**Cell counts.** Motor neuron survival was assessed by directly counting all cells displaying intact neurites longer than 4 cells in diameter following immunostaining against p75^{NTR}. Counts were performed over an area of 2.76cm² in the center of 35 mm dishes or in an area measuring 0.90 cm² along a diagonal in 24-well plates (Cassina et al., 2002). The mean density of motor neurons in control cocultures was 90±4 cells/cm².

**Immunofluorescence.** Astrocyte-motor neuron co-cultures in Lab-Tek (Nunc) slides were fixed on ice with 4% paraformaldehyde plus 0.1% glutaraldehyde in PBS. Briefly, cultures were permeabilized with 0.1% Triton X-100 in PBS for 15 min and blocked for 2 h with 10% goat serum, 2% BSA, and 0.1% Triton X-100 in PBS. Primary antibodies diluted in blocking solution were incubated overnight at 4°C. After washing with PBS, fluorophore-conjugated anti-rabbit or anti-mouse secondary antibodies diluted in blocking solution were incubated for 1 h at room temperature. The slides were mounted using ProLong antifade kit (Molecular Probes, Eugene). Primary antibodies used were rabbit anti- p75^{NTR} polyclonal antibody (1:200; Chemicon), the supernatant from the 4D5 hybridoma obtained from the Devlopmental Studies Hybridoma Bank (USA) against Islet-112 (1:100; 17), affinity-purified rabbit polyclonal antibody to nitrotyrosine obtained from Upstate, USA (1:100; 44), and cleaved caspase-3 (1:50; Cell Signaling). Secondary antibodies were Alexa-conjugated goat anti-mouse (10 µg/ml; Molecular Probes) and Cy3-conjugated goat anti-rabbit (1:400; Jackson).

**Immunohistochemistry.** Mice were transcardially perfused with 0.9% saline followed by 4% paraformaldehyde in PBS under pentobarbital deep anesthesia. The spinal cords were removed, post-fixed and paraffin-embedded. The blocks were sectioned at 5 µm thickness on a microtome. Following deparaffinization, tissue sections were preincubated at -20°C with 0.3% hydrogen peroxide in methanol. After being washed with PBS, the tissue sections were permeabilized and blocked as described above. Primary antibodies were anti-NGF-β polyclonal (1:250; Chemicon AB1526SP) or monoclonal (1:250; Chemicon MAB5260Z), rabbit anti-p75^{NTR} polyclonal (1:200, Chemicon; or 1:150, Advanced Targeting Systems) and anti-nitrotyrosine polyclonal (1:100; Ye et al., 1996). Primary antibodies were diluted in blocking solution and incubated overnight at 4oC. The secondary antibodies used were the DAKO EnVision Kit for NGF and p75^{NTR} and biotinylated goat anti-rabbit (Gibco) followed by horseradish peroxidase-conjugated streptavidin (Gibco) for nitrotyrosine. Development was performed with 0.5 mg/ml DAB solution and 0.005% (v/v) hydrogen peroxide in 0.05 M Tris-Hcl (pH 7.4). The slides were counterstained with hematoxylin. Controls were performed by omitting the primary antibody.

**Determination of NGF/proNGF.** The NGF protein concentration in the culture medium from astrocytes or spinal cord extracts was quantified using the NGF Emax ImmunoAssay System kit (Promega) following the manufacturer's instructions.

For Western blot analysis, lumbar spinal cords were homogenized in lysis buffer containing 2mM EDTA, 1% SDS, 1mM PMSF, 10 µg/ml aprotinin, 1µg/ml leupeptin and 0.5mM sodium vanadate. The samples were prepared in Laemmli buffer supplemented with 20 mM DTT and 100 mM iodoacetamide. SDS-PAGE was performed using 15% polyacrylamide gels and proteins were transferred to nitrocellulose membrane (Amersham). Membranes were blocked for 2 h in blocking buffer (5% BSA, 0.1% Tween 20 in Tris-buffered saline (TBS), pH 7.4), followed by an overnight incubation with the primary antibody diluted in blocking buffer. After washing with 0.1 % Tween in TBS, the membrane was incubated with peroxidase-conjugated goat anti-rabbit antibody (1:4000; Biorad) for 1 h, then washed and developed using the ECL chemiluminescent detection system (Amersham). Primary antibodies used were anti-NGF-β polyclonal (1:3000; Chemicon) and polyclonal antibody to pre-pro-domain of NGF (1:2,000 for #421 and 1:1500 for #418 from Pro-Hormone Sci., Los Angeles).

For immunoprecipitations, culture media from astrocyte monolayers treated under serum-free conditions were concentrated (~10X) in Centricon filters YM-3 (Millipore). Conditioned media were cleared by incubation with protein Asepharose beads (Sigma) for 1 h at 4°C. Immunoprecipitations were performed by adding 2 µg of anti-NGF polyclonal antibody (Santa Cruz Biotech.) and incubating overnight at 4°C. Protein A-Sepharose was then added and incubation continued for an additional 3 h. The beads were collected by centrifugation and washed three times with ice cold immunoprecipitation buffer (0.1% Triton X-100, 0.5% NP-40, 140 mM NaCl, protease inhibitor cocktail (Sigma), 0.025% sodium azide and 10 mM Tris-HCl, pH 8.0) and once with 10 mM Tris-HCl, pH 8.0. Immunoprecipitates were eluted from the Sepharose beads with Laemmli buffer, supplemented as described above, and the samples were boiled for 3 min before analysis by Western blot. Samples immunoprecipitated with non-immune rabbit IgG showed no bands corresponding to NGF.

**Statistical analysis.** Data analysis was performed using standard statistical packages (SigmaStat System and JMP). All values are the mean of at least 3 independent experiments performed in duplicate. To determine whether differences between treatment groups in cell cultures were significant (p<0.05), we used one- and two-way ANOVA followed by contrasts. Student's t test was used to evaluate ELISA results.

**Systemic immunization against NGF on ALS-like disease progression and survival in transgenic mouse overexpressing mutant G93A-SOD1 gene.** Animals. The transgenic mouse line overexpressing mutant G93A-SOD1 gene [TgN(SOD1-G93A)1Gur, a high expression mouse line] and the mating pairs were purchased from Jackson laboratory (Bar Harbor, ME, USA). Mice were housed and bred as described previously (Gumey et al. 1994) in accordance with the Institutional Animal Care guidelines.

Immunization protocol. Adult, female, 40-50 day-old mice, initially weighing 18-24 g, were used for these experiments. Mice were group-housed 5 mice per cage and allowed free access to food and water. For testing, a total of 10 mice per group were autoimmunized against NGF. Each received two subcutaneous injection of 25 µg of 2.5S mouse NGF (Harlam, USA) in 0.1 ml of a suspension of aluminum phosphate used as adjuvant. A second injection of 2.5S NGF (50 µg) in the same adjuvant was given 3 weeks later. Non-transgenic and vehicle-injected mice served as control animals for survival tests.

Determination of NGF antibodies present in the serum was performed after bleeding the mice before and after 3-5 and 10 weeks after NGF autoimmunization. Serum levels of anti-NGF IgG were measured using an enzyme-linked immunoassay ELISA. Multiwell plates were coated overnight at room temperature with 1 mg/ml 2.5S mouse NGF in 200 mM sodium carbonate buffer pH 9.6. After washing 3 times with 0.1 M phosphate-buffered saline PBS plus 0.05% Tween PBS-Tween, non-specific binding was blocked with bovine serum albumin 0.5 mg/ml in PBS-Tween for 1 h. After 3 more washes with PBS-Tween, test sera were applied at dilutions between 1:2000 and 1:48 000 in PBS-Tween for 1 h. The plates were washed and 100 ml of 1:1000 dilution of goat anti-rat IgG conjugated with horseradish peroxidase was added to each well for 1 h at 37°C. After 3 more washes in PBS-Tween, 100 ml of 0.04% *o*-phenylen-ediamine in phosphate-citrate buffer pH 5.0 containing 0.012% hydrogen peroxide was added to each well. The reaction was stopped by adding 50 ml of 2.5 N sulfuric acid. The reaction product was measured by determining the absorption at 492 nm.

Analysis of ALS-like disease progression and survival in transgenic mouse. Mice were examined daily for paralysis, disease progression and survival analysis. The initial symptoms of hind leg paralysis or failure to remain suspended in an inverted grid were considered as the disease progression threshold. Mice were killed at terminal stage, *i.e.* severely paralysis and inability to seek food and water.

### Results

**Increased levels of NGF immunoreactivity in the spinal cord of G93A SOD-1 transgenic mice.** The neuropil of the ventral spinal cord of 90-day-old symptomatic G93A mice (Gurney et *al*., 1994) showed a strong NGF immunoreactivity which was not present in non-transgenic littermates. In particular, large (15-25 µm) ramified non-neuronal cells, which were morphologically similar to reactive astrocytes, showed intense NGF immunoreactivity (Fig. 1A). Astrocyte processes displaying NGF immunoreactivity extended to wrap-around vacuoles characteristic of the ventral spinal cord in transgenic mice carrying SOD-ALS (45) (Fig 1A) and colocalized with fibrous-shaped astrocytes expressing GFAP (Fig 5). Immunoreactivity for p75^{NTR} and nitrotyrosine was observed only in symptomatic mice, where it was localized mainly in large degenerating motor neurons; no such neuronal immunoreactivity for p75^{NTR} or nitrotyrosine was found in non-transgenic littermates (Fig. 1A). ELISA analysis revealed that NGF levels in the lumbar spinal cord of 90-day-old symptomatic G93A mice were approximately double those seen in their *non*-transgenic littermates (Fig 1B). Western blot analyses of the lumbar spinal cord lysates showed only a slight rise in the levels of mature NGF (13kDa) but a more noteworthy increase in the 19-21, 28 and 32 kDa, but not in the 43 and 50kDa high molecular weight NGF proforms in the spinal cord of symptomatic transgenic mice (Fig 1C).

**Secretion of NGF pro-forms by reactive astrocytes induced motor neuron death.** Activation of spinal cord astrocytic cultures with either LPS (1 µg/ml) or a brief exposure to peroxynitrite (0.5 mM) increased the secretion of NGF to the culture medium by approximately 6- and 9- fold, respectively, 24 hours after treatment (Fig 2A). The concentration of NGF in the conditioned culture media was still 5-fold higher in peroxynitrite-treated cultures than in controls after 3 days. The increased concentrations of NGF in the culture media were due to an augmented release of the 21, 28 and 32 kDa pro-forms (Fig. 2B). In contrast, the 13kDa mature NGF was only weakly detected in the culture media and showed no apparent changes in either condition (Fig. 2B). Untreated astrocyte monolayers provided sufficient trophic support for motor neurons to survive in the absence of exogenous trophic factors. In contrast, astrocytes incubated with LPS or peroxynitrite become reactive, triggering a significant reduction in motor neuron survival by 35-40% over the following 72 h (p < 0.05, Fig. 2C). Two different sets of inactivating antibodies against NGF and p75^{NTR} prevented motor neuron loss induced by reactive astrocytes, while non-immune serum had no appreciable effect (Fig. 2C).

**NGF-induced motor neuron apoptosis.** Pre-incubation of the astrocyte monolayer with NGF had no effect on motor neuron survival when NGF was removed before neuron plating (Fig. 3A). In contrast, incubation with NGF reduced in a dose-dependent manner the survival of motor neurons cultured on unstimulated astrocytes (Fig. 3A). Approximately 50% of motor neurons in NGF-treated co-cultures (100 ng/ml) exhibited fewer neurites with less branching and were immunoreactive for nitrotyrosine and cleaved caspase-3 after 24 h in culture (Fig. 3B).

NGF-induced neuronal apoptosis was reduced in the presence of two different blocking antibodies to p75^{NTR}, while non-immune serum was devoid of effect. The caspase inhibitors DEVD-fmk and VAD-fmk also prevented NGF-induced motor neuron death (Fig. 3C).

Nitric oxide was required for p75^{NTR}-dependent motor neuron apoptosis. Unstimulated astrocyte cultures produced significant amounts of nitric oxide, as suggested by the accumulation of nitrite/nitrate in the culture media (4.5±2.0 µM) over a period of 72 h. Inhibition of nitric oxide production by the general NOS inhibitor L-NAME (1 mM), the selective neuronal NOS inhibitors TRIM (10 µM) and NPLA (10 µM), or the selective inducible NOS inhibitors aminoguanidine (50 µM) and LNIL (10 µM) significantly prevented the motor neuron apoptosis induced by NGF. In addition, the antioxidant urate (200 µM) also prevented the effects of NGF on motor neurons in co-culture (Fig. 3D).

To determine whether nitric oxide was directly responsible for rendering motor neurons vulnerable to NGF, the effect of NGF on pure motor neuron cultures was examined. NGF (100 ng/ml) had no effect on the survival of motor neurons maintained with glial-derived neurotrophic factor (GDNF). However, the production of low steady state concentrations of nitric oxide (<50nM) from 10µM NOC-18 was sufficient to induce motor neuron apoptosis by NGF (Fig. 4A). Nitric oxide alone did not affect motor neuron survival as previously reported (Estevez *et al.,* 1998, Raoul *et al.,* 2002).

**Apoptotic activity in culture media and spinal cord extracts.** Culture media from activated astrocytes and spinal cord extracts from G93A SOD mice (0.5 µg/ml) induced apoptosis in pure motor neuron cultures only in the presence of low steady state concentrations of nitric oxide (Fig. 4B-C). This effect was significantly prevented by the addition of antibodies that block NGF and p75^{NTR} activation. In contrast, media from control astrocytes or spinal cord extracts from non-transgenic littermates failed to induce neuronal death under identical experimental conditions (Fig 4B-C).

**Effect of NGF autoimmunization on paralysis onset and survival in G93A transgenic mice.** All NGF autoimmunized mice gained weight and did not display any signs of discomfort. Behavioral tests were conducted weekly from week 10 to determine the motor performance. NGF autoimmunization resulted in variable serum anti-NGF IgG titer levels ranging from ELISA absorption values: of 1:2000-1:5000. No anti-NGF IgG was found in samples obtained from mice injected with adjuvant only. Survival of autoimmunized mice was significantly delayed by 10-15 days in average, as compared with mice injected with adjuvant only (Fig 6). Such a delay in survival is comparable in extend to the protection exerted by other experimental treatments previously tested in G93A, including non-steroid-antiinflinflamatories, neuroproctive drugs, and antioxidants. In addition, disease onset was also delayed in most of the NGF autoimmunized mice. These results strongly indicate that production of blocking antibodies for NGF leads to a protective effect on neurodegeneration and ALS like symptoms.

### Discussion

Motor neurons are commonly thought to be unresponsive to NGF because they lack the specific TrkA receptor. However, induction of p75^{NTR} under pathological conditions may render these cells vulnerable to NGF-induced apoptosis. The present invention provides evidence that reactive astrocytes occurring in the spinal cord of symptomatic G93A mice produce NGF in sufficient concentrations to stimulate p75-dependent apoptosis in cultured motor neurons.

NGF immunoreactivity localized mainly in reactive astrocytes and correlated with p75 expression and nitrotyrosine staining of neighboring motor neurons.

Remarkably, both mature and precursor forms of NGF are induced in the lumbar spinal cords of symptomatic G93A mice, though the extent of induction is greater for the NGF pro-forms corresponding to 19-21, 28, and 32 kDa than for mature NGF. Taken together, these results show for the first time that proNGF up-regulation in activated astrocytes may play a pathogenic role in ALS.

Activation with peroxynitrite or LPS caused cultured spinal cord astrocytes to up-regulate the secretion of NGF-like species by 6-9 fold. Immunoprecipitation and Western blot analysis of conditioned media from both control and reactive astrocytes detected only minimal secretion of mature NGF but a far more robust secretion of NGF pro-forms, suggesting that astrocytes mainly secrete pro-NGF in culture. Using different experimental paradigms, the present invention provides direct evidence that NGF-like species contribute to p75-dependent motor neuron apoptosis *in* *vitro.* In co-culture experiments, it is further shown that reactive astrocyte-induced motor neuron apoptosis is prevented by antibodies that block NGF activity or antagonize p75^{NTR} activation. Moreover, a p75^{NTR}-dependent mechanism allowed exogenous NGF to induce motor neuron apoptosis in co-cultures. Only a subset of motor neurons (~40%) proved vulnerable to NGF in culture, a finding which accords with the 30-50% motor neuron loss induced by NGF in spinal cord explants (Sedel *et al*., 1999) and by Fas ligand in purified motor neuron cultures (Raoul *et al*., 2002). Finally, culture media from reactive astrocytes or extracts from degenerating spinal cords displayed neuronal pro-apoptotic activity in pure motor cultures strikingly similar to exogenous NGF, which was abolished by blocking antibodies to NGF or p75^{NTR}. Because endogenous mature NGF were found in only extremely low concentrations in the tissue extracts and nearly failed to meet the detection limit in culture media, NGF precursors (19-21, 28 and 32 kDa) are the more likely mediators of apoptosis for p75^{NTR}-expressing motor neurons. Recently, a 32 kDa species was shown to be the predominant form of NGF after spinal cord injury and to have pro-apoptotic activity on cultured p75^{NTR}-expressing oligodendrocytes (Beattie *et al*., 2002).

The inventors and others have previously shown that execution of motor neuron apoptosis requires endogenous nitric oxide and peroxynitrite formation independently of the death stimuli (Cassina *et al*., 2002, Estevez *et al*., 1998; Estevez *et al*., 1999, Raoul *et al*., 2001). NGF-induced motor neuron apoptosis is not the exception, since it was prevented by specific neuronal NOS inhibitors (nitric oxide synthetase) as well as urate, a competitive inhibitor of tyrosine nitration by peroxynitrite (Hooper et al., 1998). However, NGF-induced apoptosis was also prevented by specific inhibition of inducible NOS (nitric oxide synthase), which is mainly expressed by reactive astrocytes (Sasaki *et al.,* 2000, Cassina *et al.,* 2002), suggesting that astrocytic nitric oxide plays a role in p75^{NTR}-dependent motor neuron apoptosis. Further support for this hypothesis was provided by experiments using pure motor neuron cultures lacking astrocytes as a source of nitric oxide. Under these conditions, motor neurons strongly expressing p75^{NTR} were not sensitive to exogenous NGF. However, a low flux of nitric oxide provided by NOC-18 (<50 nM) rendered motor neurons vulnerable to NGF. Thus, increased production of both NGF and nitric oxide seems to be required for motor neurons to undergo apoptosis. These results may help to explain some of the disparate effects of NGF and p75 on neuronal survival and death. In adult motor neurons, p75^{NTR} expression seems to be linked to neuronal injury or disease. Motor neurons both in ALS patients (Kerkhoff *et al*., 1991, Seeburger *et al.*, 1993, Lowry *et al*., 2001) and in rodents which have suffered nerve injury express p75^{NTR} (Koliatsos *et al*., 1991; Rende *et al*., 1995, Ferri *et al*., 1998), and p75^{NTR} has been implicated in motor neuron death induced by axotomy (Ferri *et al*., 1998, Lowry *et al*., 2001, Wiese *et al*., 1999). These data suggest that motor neurons expressing p75^{NTR} may become vulnerable to proNGF secreted by surrounding activated astrocytes, and that this mechanism may contribute to the progressive death of motor neurons in ALS.

### Bibliography

Alexianu ME, Kozovska M, Appel SH.Immune reactivity in a mouse model of familial ALS correlates with disease progression. Neurology 2001;57:1282-1289

Beach TG, Walker R, McGeer EG. Patterns of gliosis in Alzheimer's disease and aging cerebrum. Glia. 1989;2(6):420-36.

Beattie MS, Harrington AW, Lee R, Kim JY, Boyce SL, Longo FM, Bresnahan JC, Hempstead BL, Yoon SO. ProNGF induces p75-mediated death of oligodendrocytes following spinal cord injury. Neuron. 2002; 36: 375-386.

Beckman JS.Nitric oxide and superoxide contribute to motor neuron apoptosis induced by trophic factor deprivation. J Neurosci. 1998 18:923-31.

Berkemeier LR, Winslow JW, Kaplan DR, Nikolios K, Goeddel DV, Rosenthal A. Neurotrophin-5: a novel neurotrophic factor that activates trk and trkB. Neuron. 1991 Nov; 7(5):857-66.

Bradshaw RA, Blundell TL, Lapatto R, McDonald NQ, Murray-Rust J. Nerve growth factor revisited. Trends Biochem Sci. 1993 Feb; 18(2):48-52.

Brann AB, Tcherpakov M, Williams IM, Futerman AH, Fainzilber M Nerve growth factor-induced p75-mediated death of cultured hippocampal neurons is age-dependent and transduced through ceramide generated by neutral sphingomyelinase. J Biol Chem. 2002; 277: 9812-9818.

Brooks, B. EI Escorial World Federation of Neurology criteria for the diagnosis of Amyotrophic Lateral Sclerosis. J. Neurol. Sci. 1994; 124: 96-107.

Bruijn LI, Becher MW, Lee MK, Anderson KL, Jenkins NA, Copeland NG, Sisodia SS, Rothstein JD, Borchelt DR, Price DL, Cleveland DW ALS-linked SOD1 mutant G85R mediates damage to astrocytes and promotes rapidly progressive disease with SOD1-containing inclusions. Neuron 1997; 18:327-338.

Cassina P, Peluffo H, Pehar M, Martinez-Palma L, Ressia A, Beckman JS, Estevez AG, Barbeito. Peroxynitrite triggers a phenotypic transformation in spinal cord astrocytes that induces motor neuron apoptosis. J Neurosci Res. 2002; 67:21-29.

Chang A, Nishiyama A, Peterson J, Prineas J, Trapp BD. NG2-positive oligodendrocyte progenitor cells in adult human brain and multiple sclerosis lesions. J Neurosci. 2000; 20: 6404-6412.

Crutcher KA, Scott SA, Liang S, Everson WV, Weingartner J. Detection of NGF-like activity in human brain tissue: increased levels in Alzheimer's disease. J Neurosci. 1993 Jun;13(6):2540-2550.

Danton GH, Dietrich WD. Inflammatory mechanisms after ischemia and stroke. J Neuropathol Exp Neurol. 2003; 62: 127-136.

Dechant G, Barde YA. The neurotrophin receptor p75(NTR): novel functions and implications for diseases of the nervous system. Nat Neurosci. 2002; 5: 1131-1136.

Eddleston M, Mucke L. Molecular profile of reactive astrocytes-implications for their role in neurologic disease. Neuroscience. 1993; 54: 15-36 eds. Turner, A. J. & Brachelard, H. S. (IRL Press Oxford, Washington, D.C.).

Estévez, A. G., Crow, J. P., Sampson, J. B., Reiter, C., Zhuang, Y., Richardson, G.J., Tarpey, M.M., Barbeito, L. and Beckman, J.S. Induction of nitric oxide-dependant apoptosis in motor neurons by zinc-deficient superoxide dismutase, Science, 286 (1999) 2486-2500.

Fahnestock M, Michalski B, Xu B, Coughlin MD. The precursor pro-nerve growth factor is the predominant form of nerve growth factor in brain and is increased in Alzheimer's disease. Mol Cell Neurosci. 2001; 18: 210-220.

Fahnestock M, Scott SA, Jette N, Weingartner JA, Crutcher KA. Nerve growth factor mRNA and protein levels measured in the same tissue from normal and Alzheimer's disease parietal cortex. Brain Res Mol Brain Res. 1996; 42: 175-178.

Ferri CC, Moore FA, Bisby MA. Effects of facial nerve injury on mouse motoneurons lacking the p75 low-affinity neurotrophin receptor. J Neurobiol. 1998; 34:1-9.

Friedman WJ. Neurotrophins induce death of hippocampal neurons via the p75 receptor. J Neurosci. 2000; 20: 6340-6346.

Gall C, Murray K, Isackson PJ. Kainic acid-induced seizures stimulate increased expression of nerve growth factor mRNA in rat hippocampus. Brain Res Mol Brain Res. 1991; 9:113-123.

Gurney, M.E., Pu, H., Chiu, A.Y., Dal Canto, M.C., Polchoe, C.Y., Alexander, D.D., Caliendo, J., Hentati, A., Kwon, Y.W., Deng, H.-X., Chen, W., Zhai, P., Sufit, R.L. & Siddique, T. Motor neuron degeneration in mice that express a human Cu,Z, superoxide dismutase mutation. Science 1994; 264: 1772-1775.

Gurney, M.E., Pu, H., Chiu, A.Y., Dal Canto, M.C., Polchoe, C.Y., Alexander, D.D., Caliendo, J., Hentati, A., Kwon, Y.W., Deng, H.-X., Chen, W., Zhai, P., Sufit, R.L. & Siddique, T. Motor neuron degeneration in mice that express a human Cu,Z, superoxide dismutase mutation. Science 1994; 264: 1772-1775.

Hallbook F, Ibanez CF, Persson H. Evolutionary studies of the nerve growth factor family reveal a novel member abundantly expressed in Xenopus ovary. Neuron. 1991 May;6(5):845-58.

Hempstead BL. The many faces of p75NTR. Curr Opin Neurobiol. 2002; 12: 260-267.

Henderson, C.E., Bloch-Gallego, E. & Camu, W. (1995) En: *Neural Cell Culture: A Practical Approach* (J. Cohen & G. Wilkin, eds). IRL Press, Oxford, Inglaterra. pp. 69-81.

Hirano A. Neuropathology of ALS: an overview. Neurology. 1996; 47:S63-66

Hock C, Heese K, Hulette C, Rosenberg C, Otten U. Region-specific neurotrophin imbalances in Alzheimer disease: decreased levels of brain-derived neurotrophic factor and increased levels of nerve growth factor in hippocampus and cortical areas. Arch Neurol. 2000a; 57: 846-851.

Hock C, Heese K, Muller-Spahn F, Huber P, Riesen W, Nitsch RM, Otten U. Increased CSF levels of nerve growth factor in patients with Alzheimer's disease. Neurology. 2000b; 54:2009-2011.

Hock C, Heese K, Muller-Spahn F, Hulette C, Rosenberg C, Otten U. Decreased trkA neurotrophin receptor expression in the parietal cortex of patients with Alzheimer's disease. Neurosci Lett. 1998; 241: 151-154.

Hohn A, Leibrock J, Bailey K, Barde YA. Identification and characterization of a novel member of the nerve growth factor/brain-derived neurotrophic factor family. Nature. 1990 Mar 22;344(6264):33-41.

Hooper, D. C., Spitsin, S., Kean, R. B., Champion, J. M., Dickson, G. M.,Chaudhry, I. & Koprowski, H. (1998) *Proc Natl Acad Sci USA* **95**, 675-680.

Howland DS, Liu J, She Y, Goad B, Maragakis NJ, Kim B, Erickson J, Kulik J, DeVito L, Psaltis G, DeGennaro LJ, Cleveland DW, Rothstein JD. Focal loss of the glutamate transporter EAAT2 in a transgenic rat model of SOD1 mutant-mediated amyotrophic lateral sclerosis (ALS). Proc Natl Acad Sci U S A. 2002; 99:1604-1609.

Huang et Reichardt, *Annu Rev Neurosci,* **24**: 677-736, 2001

Kerkhoff, H., Jennekens, F. G., Troost, D. & Veldman, H. (1991) *Acta Neuropathol (Berl)* **81**, 649-656.

Koliatsos VE, Crawford TO, Price DL. Axotomy induces nerve growth factor receptor immunoreactivity in spinal motor neurons. Brain Res. 1991 May 24;549(2):297-304

Kowalska A. Molecular genetics of frontotemporal dementia and parkinsonism linked to chromosome 17 (FTDP-17). Folia Neuropathol. 2002;40(3):111-118.

Kushner PD, Stephenson DI, Wright S Reactive astrogliosis is widespread in the subcortical white matter of amyotrophic lateral sclerosis brain. J. Neuropathol. Exp. Neurol. 1991; 50:263-277.

Kust BM, Copray JC, Brouwer N, Troost D, Boddeke HW. Elevated levels of neurotrophins in human biceps brachii tissue of amyotrophic lateral sclerosis. Exp Neurol. 2002; 177: 419-427.

Lefrancois T, Fages C, Brugere-Picoux J, Tardy M.Astroglial reactivity in natural scrapie of sheep. Microb Pathog. 1994; 17: 283-289.

Leibrock J, Lottspeich F, Hohn A, Hofer M, Hengerer B, Masiakowski P, Thoenen H, Barde YA. Molecular cloning and expression of brain-derived neurotrophic factor. Nature. 1989 Sep 14;341(6238):149-52.

Levi-Montalcini R, Skaper SD, Dal Toso R, Petrelli L, Leon A. Nerve growth factor: from neurotrophin to neurokine. Trends Neurosci. 1996; 19:514-520.

Liberski PP, Bratosiewicz-Wasik J, Gajdusek DC, Brown P.Ultrastructural studies of experimental scrapie and Creutzfeldt-Jakob disease in hamsters. II. Astrocytic and macrophage reaction towards axonal destruction. Acta Neurobiol Exp. 2002; 62:131-139.

Lorez H, Keller F, Ruess G, Otten U. (1989) Nerve growth factor increases in adult rat brain after hypoxic injury. Neurosci Lett. 1989; 98:339-344.

Lowry KS, Murray SS, McLean CA, Talman P, Mathers S, Lopes EC, Cheema SS.A potential role for the p75 low-affinity neurotrophin receptor in spinal motor neuron degeneration in murine and human amyotrophic lateral sclerosis. Amyotroph Lateral Scler Other Motor Neuron Disord. 2001; 2: 127-134.

Lowry, K. S., Murray, S. S., Coulson, E. J., Epa, R., Bartlett, P. F., Barrett, G.& Cheema, S. S. (2001) *J Neurosci Res* **64**,11-17.

Maisonpierre PC, Belluscio L, Squinto S, Ip NY, Furth ME, Lindsay RM, Yancopoulos GD. Neurotrophin-3: a neurotrophic factor related to NGF and BDNF. Science 1990 Mar 23;247(4949 Pt1):1446-51.

Massaro AR, Sbriccoli A, Tonali P.Reactive astrocytes within the acute plaques of multiple sclerosis are PSA-NCAM positive. Neurol Sci. 2002;23: 255-256

McDonald NQ, Blundell TL. Crystallization and characterization of the high molecular weight form of nerve growth factor (7S NGF). J Mol Biol. 1991 Jun 20;219(4):595-601.

McDonald NQ, Lapatto R, Murray-Rust J, Gunning J, Wlodawer A, Blundell TL. New protein fold revealed by a 2.3-A resolution crystal structure of nerve growth factor. Nature. 1991 Dec 5; 354(6352):411-4.

McGeer PL, Akiyama H, Itagaki S, McGeer EG Immune system response in Alzheimer's disease. Can J Neurol Sci. 1989 Nov;16(4 Suppl):516-527.

Miller et al., J Mol Biol 48: 443-453 (1970)

Mufson EJ, Kordower JH. Cortical neurons express nerve growth factor receptors in advanced age and Alzheimer disease. Proc Natl Acad Sci U S A. 1997; 89:569-573.

Mufson EJ, Lavine N, Jaffar S, Kordower JH, Quirion R, Saragovi HU. Reduction in p140-TrkA receptor protein within the nucleus basalis and cortex in Alzheimer's disease. Exp Neuro). 1997; 146: 91-103.

Needleman *et al*., J Mol Biol 48: 443-53 (1972).

Park JA, Lee JY, Sato TA, Koh JY. Co-induction of p75NTR and p75NTR-associated death executor in neurons after zinc exposure in cortical culture or transient ischemia in the rat. J Neurosci. 2000; 20: 9096-9103.

Pearson *et al*., Proc Natl Acad Sci USA 85:2444-8 (1988)

Pike CJ, Cummings BJ, Cotman CW.Early association of reactive astrocytes with senile plaques in Alzheimer's disease. Exp Neurol. 1995;132:172-179.

Raoul, C., Estévez, A. G., Nishimune, H., Cleveland, D. W., deLapeyrière, O., Henderson, C. E., Haase, G. & Pettmann, B. (2002) *Neuron* **35**, 1067-1083.

Rende, M., Giambanco, I., Buratta, M. & Tonali, P. (1995) *J Comp. Neurol.* **363**, 249-263.

Renkawek K, Stege GJ, Bosman GJ.Dementia, gliosis and expression of the small heat shock proteins hsp27 and alpha B-crystallin in Parkinson's disease. Neuroreport. 1999; 10: 2273-2276.

Richardson, G. J., Tarpey, M. M., Barbeito, L. & Beckman, J. S. (1999) *Science* **286,** 2498-2500.

Ridet JL, Malhotra SK, Privat A, Gage FH.Reactive astrocytes: cellular and molecular cues to biological function. Trends Neurosci. 1997; 20:570-577.

Rothstein JD. Excitotoxicity hypothesis. Neurology. 1996; 47:S19-25.

Roux PP, Colicos MA, Barker PA, Kennedy TE. p75 neurotrophin receptor expression is induced in apoptotic neurons after seizure. J Neurosci. 1999; 19: 6887-6896.

Saneto, R. P. & Vellis, J. D. In *Neurochemistry: a Practical Approach* 1987 (A. J. Turner & H. S. Brachelard, Eds.). IRL Press, Oxford-Washington, DC, pp. 27-63.

Sasaki S, Shibata N, Komori T, Iwata M iNOS and nitrotyrosine immunoreactivity in amyotrophic lateral sclerosis. Neurosci Lett. 2000; 291:44-48.

Sasaki S, Warita H, Abe K, Iwata M.Inducible nitric oxide synthase (iNOS) and nitrotyrosine immunoreactivity in the spinal cords of transgenic mice with a G93A mutant SOD1 gene. J Neuropathol Exp Neurol 2001; 60:839-846.

Schipper HM. Astrocytes, brain aging, and neurodegeneration. Neurobiol Aging. 1996 May-Jun;17(3):467-480.

Scott J, Selby M, Urdea M, Quiroga M, Bell Gl, Rutter WJ. Isolation and nucleotide sequence of a cDNA encoding the precursor of mouse nerve growth factor. Nature. 1983 Apr 7; 302(5908):538-40.

Sedel F, Bechade C, Triller A. Nerve growth factor (NGF) induces motoneuron apoptosis in rat embryonic spinal cord in vitro. Eur J Neurosci. 1999;11:3904-3912.

Seeburger JL, Tarras S, Natter H, Springer JE. Spinal cord motoneurons express p75NGFR and p145trkB mRNA in amyotrophic lateral sclerosis. Brain Res. 1993; 621: 111-115.

Smith et al., J Theor Biol 91: 379-380 (1981).

Troy CM, Friedman JE, Friedman WJ. Mechanisms of p75-mediated death of hippocampal neurons. Role of caspases. J Biol Chem. 2002; 277:.34295-34302.

Ullrich A, Gray A, Berman C, Dull TJ. Human beta-nerve growth factor gene sequence highly homologous to that of mouse. Nature, 1983 Jun 30; 303(5920):821-5.

Wiese, S., Metzger, F., Holtmann, B. & Sendtner, M. (1999) *Eur. J. Neurosci.* **11**, 1668-1676.

Ye, Y. Z., Strong, M., Huang, Z. Q. & Beckman, J. S. (1996) *Methods Enzymol* **269**, 201-209.

## Claims

1. Use of a composition capable of inhibiting *in vivo* the binding of proapoptotic neurotrophin to p75^{NTR} receptor expressed by neuronal or glial cell, in the preparation of a medicament for inhibiting neuronal or glial cell apoptosis caused by neuroinflammation in a mammal.

2. The use according to Claim 1, wherein said neuroinflammation is associated with a neurodegenerative disease.

3. Use of a composition comprising an effective amount of an immunogenic compound which is capable of inducing an immune response against a proapoptotic neurotrophin, or an effective amount of a hapten combined with appropriate carriers and/or adjuvants to render the resulting combination capable of inducing an immune response against a proapoptotic neurotrophin, in the preparation of a medicament for the treatment of neurodegenerative disease associated with neuroinflammation.

4. The use according to Claim 3, wherein said immunogenic compound or hapten comprises a neurotrophin or a fragment of a neurotrophin which can be rendered immunogenic when combined with appropriate carriers and/or adjuvants, or a derivative of said neurotrophin fragment, retaining substantially the same immunological properties as the native neurotrophin or the native fragment of neurotrophin.

5. The use according to Claim 4, wherein said neurotrophin fragment or derivative thereof is effective to induce antibodies directed to a neoepitope that is not present on the native forms of neurotrophin but is revealed by proteolytic cleavage of the native form of neurotrophin.

6. The use according to Claim 4 or 5, wherein said derivative is a neurotrophin or a fragment of a neurotrophin which is modified by amino acid substitution, deletion and/or addition.

7. The use according to Claim 6, wherein said amino acid substitution, deleletion and/or addition does not affect the tertiary structure of the modified neurotrophin or neurotrophin fragment as compared to the native one from which it derives.

8. The use according to Claim 6, wherein said amino acid substitution, deleletion and/or addition increases the immunogenicity of the modified neurotrophin or neurotrophin fragment as compared to the native one.

9. The use according to Claim 3, wherein said immunogenic compound or hapten comprises a mature form of a neurotrophin or an immunogenic or hapten fragment thereof.

10. The use according to Claim 3, wherein said immunogenic compound or hapten comprises a precursor form of a neurotrophin or a fragment thereof, said fragment comprising at least part of its amino acid sequence which is not comprised in the corresponding mature form of the neurotrophin, such as the preprodomain of NGF.

11. The use according to any of Claims 3-10, wherein the neurotrophin is selected among the group consisting of NGF, BDNF, NT-3 and NT-4, pro-NGF, pro-BDNF.

12. The use according to any of Claims 3-11, wherein said immunogenic compound or hapten is a peptide or polypeptide comprising at least 6 consecutive amino acids of one the following sequences:
a. an immunogenic or hapten fragment of any of SEQ ID NOs 1-4,
b. an immunogenic or hapten fragment of SEQ ID NO:1 comprising one of the following sequences: IKGKE (SEQ ID NO:5), CRGIDSKHW (SEQ ID NO:6), GKQA (SEQ ID NO:7) and SRKAV (SEQ ID NO:8),
c. an immunogenic or hapten fragment of SEQ ID NO:2 comprising one of the following sequences: MSGGT (SEQ ID NO:9), CRGIDKRHW (SEQ ID NO:10), SKKRI (SEQ ID NO:11), TIKRG (SEQ ID NO:12),
d. an immunogenic or hapten fragment of SEQ ID NO:3 comprising one of the following sequences: IRGHQ (SEQ ID NO:13), CRGIDDKHW (SEQ ID NO:14), NNKLV (SEQ ID NO:15), SRKIG (SEQ ID NO:16),
e. an immunogenic or hapten fragment of SEQ ID NO:4 comprising one of the following sequences: LRGRE (SEQ ID NO:17), CRGVDRRHW (SEQ ID NO:18), AQGRV (SEQ ID NO:19), LSRTG (SEQ ID NO:20).
f. a peptide exhibiting at least 70% identity, preferably 80% identity and more preferably 90% identity with one of the immunogenic or hapten fragment defined in a.-e., said peptide retaining the same immunological properties as the native one from which it derives.

13. The use according to any of Claims 3-12, wherein said hapten is coupled to a carrier molecule to render the resulting coupled molecule, immunogenic.

14. The use according to Claim 13, wherein said carrier molecule is selected among the group consisting of bovine serum albumine, immunoglobulin, thyroglobulin, ovalbumin, tetanus toxoid, keyhole limpet hemocyanin or lipid moieties.

15. The use according to any of Claims 1 or 2, wherein said compound binds to a proapoptotic neurotrophin or p75 ^{NTR} receptor, thereby blocking the interaction between said proapoptotic neurotrophin and p75^{NTR}.

16. The use according to Claim 15, wherein the neurotrophin is selected among the group consisting of NGF, BDNF, NT-3 and NT-4, pro-NGF and pro-BDNF.

17. The use according to any of Claims 15-16, wherein said compound is an antagonist of said neurotrophin.

18. The use according to Claim 17, wherein said antagonist is a fragment of said neurotrophin or a derivative thereof having at least 70% identity, preferably at least 80% identity and more preferably, at least 90% identity with the native fragment.

19. The use according to any of Claims 15-18, wherein said compound is an antibody directed against a neurotrophin, or a fragment thereof, or a derivative thereof exhibiting at least 70% identity, preferably at least 80% identity and more preferably at least 90% identity with a native fragment of said antibody, said fragment and said derivative retaining the same binding affinity towards neurotrophin as the native antibody from which it derives.

20. The use according to Claim 19, wherein said antibody is a monoclonal antibody.

21. The use according to Claim 19, wherein said antibody is contained in a polyclonal serum obtainable by immunizing a mammal with the immunogenic compound or composition defined in any of Claims 3-14.

22. The use according to any of Claims 2 to 21, wherein said neurodegenerative disease is one of the following diseases: amyotrophic lateral sclerosis, Alzheimer's disease, Parkinson's disease, Huntington's disease, Fronto temporal dementia, parkinsonism linked to chromosome 17 and prion diseases such as Kuru, Creutzfeld-Jacob disease, scrapie and bovine spongiform encephalitis.

23. An immunogenic composition for inducing an immune response against a proapoptotic neurotrophin, comprising an effective amount of the immunogenic compound or the hapten defined in any of Claims 3-14, in combination with a vehicle.

24. The immunogenic composition according to Claim 23, wherein the vehicle is appropriate for *in vivo* administration.

25. The immunogenic composition of Claim 23 or 24, wherein said effective amount is comprised between 0,5 µg and 2000 µg of said immunogenic compound or hapten.

26. The immunogenic composition of any of Claims 23-25, **characterized in that** it comprises a peptide or a polypeptide comprising at least 6 consecutive amino acids of one the following sequences:
a. an immunogenic or hapten fragment of any of SEQ ID NOs 1-4,
b. an immunogenic or hapten fragment of SEQ ID NO:1 comprising one of the following sequences: IKGKE (SEQ ID NO:5), CRGIDSKHW (SEQ ID NO:6), GKQA (SEQ ID NO:7) and SRKAV (SEQ ID NO:8),
c. an immunogenic or hapten fragment of SEQ ID NO:2 comprising one of the following sequences: MSGGT (SEQ ID NO:9), CRGIDKRHW (SEQ ID NO:10), SKKRI (SEQ ID NO:11), TIKRG (SEQ ID NO:12),
d. an immunogenic or hapten fragment of SEQ ID NO:3 comprising one of the following sequences: IRGHQ (SEQ ID NO:13), CRGIDDKHW (SEQ ID NO:14), NNKLV (SEQ ID NO:15), SRKIG (SEQ ID NO:16),
e. an immunogenic or hapten fragment of SEQ ID NO:4 comprising one of the following sequences: LRGRE (SEQ ID NO:17), CRGVDRRHW (SEQ ID NO:18), AQGRV (SEQ ID NO:19), LSRTG (SEQ ID NO:20).
f. a peptide exhibiting at least 70% identity, preferably 80% identity and more preferably 90% identity with one of the immunogenic or hapten fragment defined in a.-e., said peptide retaining the same immunological properties as the native one from which it derives,
said hapten fragment being optionnally coupled to a carrier molecule and/or combined with appropriate adjuvants to render the resulting combination immunogenic.

27. The immunogenic composition according to Claim 25, wherein said immunogenic or hapten fragment has a size from 10 to 100 amino acids, preferably, from 10 to 50 amino acids and more preferably from 20 to 30 amino acids, and is optionnally coupled to a carrier molecule or combined with appropriate adjuvants for providing an effective immune response.

28. The immunogenic composition according to any of Claims 23-26, which further comprises an aduvant.

29. The immunogenic composition according to Claim 27, wherein said adjuvant is selected among the group consisting of aluminium hydroxide, aluminium phosphate, MPL1M, QS621 or incomplete Freund's adjuvant.

30. The immunogenic composition according to any of Claims 23-28, which include a plurality of immunogenic compounds effective to induce an immune response against at least two different neurotrophin antigens.

31. A composition capable of inhibiting *in vivo* the binding of proapoptotic neurotrophin to p75^{NTR} receptor expressed by neuronal or glial cell, comprising an effective amount of an antibody directed against a proapoptotic neurotrophin or functional derivatives thereof as defined in any of Claims 19-21, in combination with an appropriate acceptable vehicle for *in vivo* administration.

32. The composition according to Claim 30, comprising a combination of antibodies that bind to at least two different neurotrophins.

33. The composition according to any of Claims 30 or 31, wherein an effective amount of antibodies corresponds to a serum amount of immunoreactivity against the target neurotrophin that is at least four times higher than a serum level of immunoreactivity against the same target neurotrophin measured in a control serum sample.

34. A composition for the prevention and/or the treatment of neurodegenerative diseases associated with neuroinflammation, comprising an effective amount of an antibody directed against a mature form of a neurotrophin, and preferably NGF or BDNF.

35. A use of an antibody directed against a mature form of a neurotrophin in the preparation of a composition for the prevention and/or the treatment of neurodegenerative diseases associated with neuroinflammation.

36. A use of an antibody directed against a neurotrophin or a neurotrophin fragment or a functional derivative thereof, in the preparation of a drug for preventing neuronal or glial cell death caused by neuroinflammation.

37. A nucleic acid encoding an immunogenic or hapten fragment as defined in Claim 26 or 27.

38. A vector comprising the nucleic acid of Claim 37 and appropriate elements for replication of said nucleic acid in a host cell and, optionnaly, expression of said nucleic acid.

39. The vector according to Claim 38, wherein said vector is capable of autonomous replication in a mammalian cell.

40. The vector according to Claim 38, wherein said vector is selected from the group consisting of a plasmid, a phage, a cosmid, a minichromosome, and a virus.

41. The vector according to Claim 38, wherein said vector is appropriate for gene therapy treatment.


SEQUENCE LISTING

<110>  INSTITUT PASTEUR
       INSTITUTO DE INVESTIGACIONES BIOLOGICAS CLEMENTE ESTABLE (IIBCE)

<120>  ACTIVE OR PASSIVE IMMUNIZATION AGAINST PROAPOPTOTIC NEUROTROPHINS FOR THE
TREATMENT AND/OR PREVENTION OF NEURODEGENERATIVE DISEASES

<130>  B5735

<140>  EP 03/291837.7
<141>  2003-07-24

<160>  20

<170>  PatentIn version 3.1

<210>  1
<211>  120
<212>  PRT
<213>  Homo sapiens

<400>  1

Ser Ser Ser His Pro Ile Phe His Arg Gly Glu Phe Ser Val Cys Asp
1               5                   10                  15

Ser Val Ser Val Asn Val Gly Asp Lys Thr Thr Ala Thr Asp Ile Lys
            20                  25                  30

Gly Lys Glu Val Met Val Leu Gly Glu Val Asn Ile Asn Asn Ser Val
        35                  40                  45

Phe Lys Gln Tyr Phe Phe Glu Thr Lys Cys Arg Asp Pro Asn Pro Val
    50                  55                  60

Asp Ser Gly Cys Arg Gly Ile Asp Ser Lys His Trp Asn Ser Tyr Cys
65                  70                  75                  80

Thr Thr Thr His Thr Phe Val Lys Ala Leu Thr Met Asp Gly Lys Gln
                85                  90                  95

Ala Ala Trp Arg Phe Ile Arg Ile Asp Thr Ala Cys Val Cys Val Leu
            100                 105                 110

Ser Arg Lys Ala Val Arg Arg Ala
        115                 120

<210>  2
<211>  126
<212>  PRT
<213>  Homo sapiens

<400>  2

His Ser Asp Pro Ala Arg Arg His Ser Asp Pro Ala Arg Arg Gly Glu
1               5                   10                  15

Leu Ser Val Cys Asp Ser Ile Ser Glu Trp Val Thr Ala Ala Asp Lys
            20                  25                  30

Lys Thr Ala Val Asp Met Ser Gly Gly Thr Val Thr Val Leu Glu Lys
        35                  40                  45

Val Pro Val Ser Lys Gly Gln Leu Lys Gln Tyr Phe Tyr Glu Thr Lys
    50                  55                  60

Cys Asn Pro Met Gly Tyr Thr Lys Glu Gly Cys Arg Gly Ile Asp Lys
65                  70                  75                  80

Arg His Trp Asn Ser Gln Cys Arg Thr Ser Gln Ser Tyr Val Arg Ala
                85                  90                  95

Leu Thr Met Asp Ser Lys Lys Arg Ile Gly Trp Arg Phe Ile Arg Ile
            100                 105                 110

Asp Thr Ser Cys Val Cys Thr Leu Thr Ile Lys Arg Gly Arg
        115                 120                 125

<210>  3
<211>  119
<212>  PRT
<213>  Homo sapiens

<400>  3

<210>  4
<211>  130
<212>  PRT
<213>  Homo sapiens

<400>  4

Gly Val Ser Glu Thr Ala Pro Ala Ser Arg Arg Gly Glu Leu Ala Val
1               5                   10                  15

Cys Asp Ala Val Ser Gly Trp Val Thr Asp Arg Arg Thr Ala Val Asp
            20                  25                  30

Leu Arg Gly Arg Glu Val Glu Val Leu Gly Glu Val Pro Ala Ala Gly
        35                  40                  45

Gly Ser Pro Leu Arg Gln Tyr Phe Phe Glu Thr Arg Cys Lys Ala Asp
    50                  55                  60

Asn Ala Glu Glu Gly Gly Pro Gly Ala Gly Gly Gly Gly Cys Arg Gly
65                  70                  75                  80

Val Asp Arg Arg His Trp Val Ser Glu Cys Lys Ala Lys Gln Ser Tyr
                85                  90                  95

Val Arg Ala Leu Thr Ala Asp Ala Gln Gly Arg Val Gly Trp Arg Trp
            100                 105                 110

Ile Arg Ile Asp Thr Ala Cys Val Cys Thr Leu Leu Ser Arg Thr Gly
        115                 120                 125

Arg Ala
    130

<210>  5
<211>  5
<212>  PRT
<213>  Homo sapiens

<400>  5

Ile Lys Gly Lys Glu
1               5

<210>  6
<211>  9
<212>  PRT
<213>  Homo sapiens

<400>  6

Cys Arg Gly Ile Asp Ser Lys His Trp
1               5

<210>  7
<211>  4
<212>  PRT
<213>  Homo sapiens

<400>  7

Gly Lys Gln Ala
1


<210>  8
<211>  5
<212>  PRT
<213>  Homo sapiens

<400>  8

Ser Arg Lys Ala Val
1               5


<210>  9
<211>  5
<212>  PRT
<213>  Homo sapiens

<400>  9

Met Ser Gly Gly Thr
1               5


<210>  10
<211>  9
<212>  PRT
<213>  Homo sapiens

<400>  10

Cys Arg Gly Ile Asp Lys Arg His Trp
1               5


<210>  11
<211>  5
<212>  PRT
<213>  Homo sapiens

<400>  11

Ser Lys Lys Arg Ile
1               5


<210>  12
<211>  5
<212>  PRT
<213>  Homo sapiens

<400>  12

Thr Ile Lys Arg Gly
1               5

<210>  13
<211>  5
<212>  PRT
<213>  Homo sapiens

<400>  13

Ile Arg Gly His Gln
1               5


<210>  14
<211>  9
<212>  PRT
<213>  Homo sapiens

<400>  14

Cys Arg Gly Ile Asp Asp Lys His Trp
1               5


<210>  15
<211>  5
<212>  PRT
<213>  Homo sapiens

<400>  15

Asn Asn Lys Leu Val
1               5


<210>  16
<211>  5
<212>  PRT
<213>  Homo sapiens

<400>  16

Ser Arg Lys Ile Gly
1               5


<210>  17
<211>  5
<212>  PRT
<213>  Homo sapiens

<400>  17

Leu Arg Gly Arg Glu
1               5


<210>  18
<211>  9
<212>  PRT
<213>  Homo sapiens

<400>  18

Cys Arg Gly Val Asp Arg Arg His Trp
1               5

<210>  19
<211>  5
<212>  PRT
<213>  Homo sapiens

<400>  19

Ala Gln Gly Arg Val
1               5


<210>  20
<211>  5
<212>  PRT
<213>  Homo sapiens

<400>  20

Leu Ser Arg Thr Gly
1               5